# EUROPÄISCHE PATENTSCHRIFT

(11) **EP 1 971 666 B1**
(45) Veröffentlichungstag und Bekanntmachung des Hinweises auf die Patenterteilung: **14.12.2011**
(21) Anmeldenummer: 07702673.0
(22) Anmeldetag: 10.01.2007
(51) Int. Cl.: C09K 11/08, C09K 11/02

(54) **VERFAHREN ZUR SYNTHESE VON NANOKRISTALLINEN LEUCHTSTOFFPARTIKELN UND GEMÄSS DIESEM VERFAHREN ERHÄLTLICHE NANOKRISTALLINE LEUCHTSTOFFPARTIKEL**
PROCESS FOR THE SYNTHESIS OF NANOCRYSTALLINE PHOSPHOR PARTICLES AND NANOCRYSTALLINE PHOSPHOR PARTICLES OBTAINABLE BY THIS PROCESS
PROCÉDÉ DE SYNTHÈSE DE PARTICULES DE SUBSTANCE LUMINESCENTE NANOCRISTALLINES ET PARTICULES DE SUBSTANCE LUMINESCENTE NANOCRISTALLINES OBTENUES SELON CE PROCÉDÉ

(30) Priorität: 11.01.2006 DE 102006001414
(43) Veröffentlichungstag der Anmeldung: 24.09.2008
(73) Patentinhaber: Karlsruher Institut für Technologie, 76131 Karlsruhe (DE)
(72) Erfinder: FELDMANN, Claus, 76275 Ettlingen (DE); BÜHLER, Gunnar, 56645 Nickenich (DE)
(74) Vertreter: Müller-Boré & Partner Patentanwälte
(86) Internationale Anmeldenummer: PCT/EP2007/000175
(87) Internationale Veröffentlichungsnummer: WO 2007/082663

(56) Entgegenhaltungen:
- US-A1- 2005 255 236
- RIWOTZKI K ET AL: "LIQUID-PHASE SYNTHESIS OF DOPED NANOPARTICLES: COLLOIDS OF LUMINESCING LAPO4:EU AND CEPO4:TB PARTICLES WITH A NARROW PARTICLE SIZE DISTRIBUTION" JOURNAL OF PHYSICAL CHEMISTRY. B, MATERIALS, SURFACES, INTERFACES AND BIOPHYSICAL, WASHINGTON, DC, US, Bd. 104, 2000, Seiten 2824-2828, XP001035003 ISSN: 1089-5647
- RIWOTZKI K; MAYSSAMY H; SCHNABLEGGER H; KORNOWSKI A; HAASE M: "Synthese von Kolloiden und redispergierbaren Pulvern stark lumineszierender LaPO4:Ce,Tb Nanokristalle" ANGEWANDTE CHEMIE, Bd. 113, Nr. 3, 2001, Seiten 574-578, XP002425778 in der Anmeldung erwähnt
- FELDMANN C: "Polyol-Mediated Synthesis of Nanoscale Functional Materials" ADVANCED FUNCTIONAL MATERIALS, Bd. 13, Nr. 2, Februar 2003 (2003-02), Seiten 101-107, XP002342330 in der Anmeldung erwähnt
- KÖMPE K; BORCHERT H; STORZ J; LOBO A; ADAM S; MÖLLER T; HAASE M: "Mit einer Quantenausbeute von 70% grün lumineszierende CePO4:Tb-Nanopartikel mit einer Schale aus LaPO4" ANGEWANDTE CHEMIE, Bd. 115, 2003, Seiten 5672-5675, XP002425779 in der Anmeldung erwähnt
- ANTONIETTI M; KUANG D; SMARSLY B; ZHOU Y: "Ionische Flüssigkeiten für die Synthese funktioneller Nanopartikel und anderer anorganischer Nanostrukturen" ANGEWANDTE CHEMIE, Bd. 116, 2004, Seiten 5096-5100, XP002425780 in der Anmeldung erwähnt

## Beschreibung

Die vorliegende Erfindung betrifft ein Verfahren zur gezielten Synthese von anorganischen Leuchtstoffpartikeln, welche einen Partikeldurchmesser von 1 bis 100 nm, eine nahezu monodisperse Größenverteilung im Bereich von ±20% und eine Quantenausbeute, in Abhängigkeit von der gewählten Materialklasse, von mindestens 20% aufweisen, gemäß diesem Verfahren erhältliche anorganische Leuchtstoffpartikel, sowie deren Verwendung zur Beschichtung auf oder zur Einbettung in ein Substrat, wobei das Substrat aus der Gruppe, bestehend aus einem Papier-Polymer-, Glas-, Metall- oder Keramiksubstrat, ausgewählt ist, beispielsweise zur Diagnostik, Therapie, als Kontrastmittel, als Markierung, Sicherheitsmerkmal, zur Beleuchtung sowie Datenspeicherung und/oder Datenvervielfältigung.

Leuchtstoffpartikel bzw. Leuchtstoffe (engl. "phosphors") finden vielfältige Anwendung in Lampen bzw. zur Beleuchtung (beispielsweise in Fluoreszenzlampen, Kompaktfluoreszenzlampen, Sonnenbanklampen, Leuchtdioden), in Bildschirmen (beispielsweise Kathodenstrahlröhren, Plasma-Anzeigeelemente, Feldemissions-Anzeigeelemente) der zur Detektion hochenergetischer Strahlung (beispielsweise in Röntgendetektoren, in Bildplatten, zur Tomographie). Dabei weisen herkömmliche Leuchtstoffpartikel üblicherweise einen Partikeldurchmesservon mehr als 1 µm auf.

Besonderes Interesse finden Leuchtstoffpartikel mit Partikeldurchmessern von weniger als 100 nm (sogenannte nanoskalige Leuchtstoffe) derzeit jedoch in Anwendungsbereichen, die mit herkömmlichen Leuchtstoffpartikeln mit Partikeldurchmessern von mehr als 1 µm nicht realisierbar sind. Dies gilt insbesondere für transparente lumineszierende Schichten oder Markierungen auf Glas-, Papier-, Metall- oder Kunststoffsubstraten (beispielsweise als Sicherheitsmerkmal auf Banknoten oder Ausweispapieren), transparente lumineszierende Füllstoffe in transparenten Matrices (beispielsweise Glas- oder Kunststoffmatrices) oder biologisch-medizinische Anwendungen, beispielsweise in der Diagnostik und Therapie von Tumorerkrankungen, der Zellmarkierung oder in Fluoreszenzresonanz-Energietransfer-Assays (FRET-Assays). Bei den vorstehend erwähnten klassischen Anwendungen im Display- bzw. Lampensektor könnten solche Leuchtstoffpartikel mit Partikeldurchmessern von weniger als 100 nm im Hinblick auf eine Materialersparnis und Miniaturisierung von Interesse werden.

Neben einer guten Verarbeitbarkeit sowie einer hohen optischen Transparenz sind eine definierte Partikelgröße mit enger Größenverteilungskurve sowie eine geringe Agglomerationstendenz wesentliche Voraussetzungen für eine technische Anwendbarkeit von Leuchtstoffpartikeln mit Partikeldurchmessern von weniger als 100 nm. Außerdem ist das Vorliegen kristalliner Materialien mit definierten Oberflächeneigenschaften bzw. definierter Oberflächenausstattung erforderlich, da nur mit solchen hohe Quantenausbeuten erzielt werden können.

Im Stand der Technik sind bisher im Wesentlichen II-VI- und III-V-Halbleiter-Quantenpunkte sowie Partikel von Seltenerd-dotierten Metallphosphaten und -vanadaten als Leuchtstoffpartikel mit Partikeldurchmessern von weniger als 100 nm bekannt (vgl. beispielsweise C.B. Murray, D.J. Norris, M.G. Bawendi, J. Am. Chem. Soc. 1993, 115, 8706; K. Riwotzki, H. Meyssamy, H. Schnablegger, A. Kornowski, M. Haase, Angew. Chem. 2001, 113, 574; C. Feldmann, Adv. Funct., Mater. 2003, 13, 101). Um dabei jedoch lumineszierende Nanokristalle höchster Qualität zu erhalten, ist zum einen die Reaktionsführung bei erhöhter Temperatur (üblicherweise im Bereich von 150°C bis 250°C) erforderlich, um die Zahl der Gitterdefekte zu minimieren. Zum anderen ist die Verwendung von stark-koordinierenden Lösungsmitteln oder Stabilisatoren während der Synthese für die Steuerung der Partikelgröße, die Kontrolle des Agglomerationsgrads, der Abschirmung der Partikeloberflächen sowie die Erhöhung der chemisch-physikalischen Stabilität wesentlich. Beispiele für geeignete, stark-koordinierende Lösungsmittel bzw. Stabilisatoren sind Polyole, wie Glycerin und Diethylenglykol, Phosphane, wie Trioctylphosphan, Phosphanoxide, Phosphate, wie Octylphosphat, Amine, wie Octylamin und Pyridin, Phosphate, Thiole, wie Dekanthiol, Thioglycerin, Carbonsäuren, wie z.B. Ölsäure, und Polymere, wie Polyvinylpyrrolidon. Eine anschließende Änderung bzw. Modifizierung der Oberflächenausstattung derart erhaltener Leuchtstoffpartikel, d.h. das Entfernen der stark-koordinierenden Lösungsmittelmoleküle oder Stabilisatoren, bringt neben den zusätzlichen Prozessschritten auch die Risiken von kolloidalem Kollaps, Agglomeration und Oberflächenzerstörung mit sich.

Zudem weisen die derart erhaltenen Leuchtstoffpartikel in der Regel Defekte oder eine amorphe Oberfläche auf, was zu einer deutlichen Verringerung der Quantenausbeute führt. Zur Erhöhung der Quantenausbeute dieser derart erhaltenen Leuchtstoffpartikel kann eine anorganische, nicht lumineszierende Hülle bzw. Schale um die Leuchtstoffpartikel unter Bildung einer Kem-Schale-Struktur vorgesehen werden (vgl. beispielsweise H. Kömpe, H. Borchert, J. Storz, A. Lobo, S. Adam, T. Möller, M. Haase, Angew. Chem. 2003, 115, 5672).

Riwotzki et al, Journal of Physical Chemistry B 2000, 104, 2824-2828, beschreibt Leuchtstoffpartikel mit Größen im Nanometerbereich, hergestellt in Tris(ethylhexyl)phosphat, Trioctylamin und kristalliner Phosphorsäure, was weder im Hinblick auf die chemische Zusammensetzung, noch im Hinblick auf die Lösungsmitteleigenschaften als ionische Flüssigkeit bezeichnet werden kann.

Wie vorstehend bereits erwähnt, werden die Leuchtstoffpartikel in herkömmlichen Syntheseverfahren in stark-koordinierenden Lösungsmitteln hergestellt, deren Entfernung nach der Synthese gar nicht oder nur durch sehr arbeitsintensive Verfahren oder nur unter partieller Agglomeration der Leuchtstoffpartikel oder unter partieller Degradation der Leuchtstoffpartikel möglich ist. Dies hat zur Folge, daß bei Verwendung von solchen stark-koordinierenden Lösungsmitteln bei der Synthese von Leuchtstoffpartikeln eine Anpassung der Oberflächenausstattung im Hinblick auf die Anforderungen einer speziellen Anwendung kaum möglich ist. Vielmehr ist die Oberflächenausstattung der Leuchtstoffpartikel von dem jeweiligen Syntheseverfahren abhängig. Zudem sind die im Stand der Technik bekannten Synthesen in der Regel zeit- und aufarbeitsaufwändig, oftmals schwierig zu reproduzieren und ergeben die Leuchtstoffpartikel häufig nur in geringen Ausbeuten.

Der vorliegenden Erfindung liegt die Aufgabe zugrunde, ein Verfahren zur gezielten Synthese von anorganischen Leuchtstoffpartikeln, die Partikeldurchmessem von weniger als 100 nm, eine definierte Partikelgröße mit enger Größenverteilungskurve, eine geringe Agglomerationstendenz sowie in Abhängigkeit von der gewählten Materialklasse hohe Quantenausbeuten aufweisen, bereitzustellen, wobei das Verfahren einfach und effizient sein soll.

Diese Aufgabe wird durch die in den Ansprüchen gekennzeichneten Ausführungsformen gelöst.

Insbesondere wird ein Verfahren zur gezielten Synthese von anorganischen Leuchtstoffpartikeln bereitgestellt, welche einen Partikeldurchmesser von 1 bis 100 nm, eine nahezu monodisperse Größenverteilung im Bereich von ±20% und in Abhängigkeit von der gewählten Materialklasse eine Quantenausbeute von mindestens 20% aufweisen, umfassend die Schritte (a) des Umsetzens mindestens eines Metallprecursors mit mindestens einem Fällungsreagens in einem ersten Lösungsmittel oder Lösungsmittelgemisch bei einer Temperatur im Bereich von -50°C bis 200°C unter Erhalten eines ersten Reaktionsgemisches, (b) des thermischen Behandeins durch Mikrowelleneinstrahlung des in Schritt (a) erhaltenen ersten Reaktionsgemisches bei einer Temperatur im Bereich von 150°C bis 700°C für eine Zeitdauer im Bereich von 5 Sekunden bis 30 Minuten in einem zweiten Lösungsmittel oder Lösungsmittelgemisch, welches mindestens eine lonische Flüssigkeit umfasst, und (c) des Isolierens und/oder Aufreinigens der Leuchtstoffpartikel.

Bei dem erfindungsgemäßen Verfahren wird zunächst in Schritt (a) mindestens ein Metallprecursor mit einem Fällungsreagenz umgesetzt. Dabei ist der mindestens eine Metallprecursor aus der Gruppe, bestehend aus Metallhalogeniden, -nitraten, -sulfaten, -hydrogensulfaten, -acetaten, -acetylacetonaten, -oxalaten, -carbonaten, -hydrogen-carbonaten, -phosphaten, -hydrogenphosphaten, -dihydrogenhosphaten, -hydriden, -alkoholaten, metallorganischen Verbindungen und Kombinationen davon ausgewählt. Dabei ist dem Fachmann bekannt, in welcher Weise ein für die Synthese von Leuchtstoffpartikeln geeigneter Metallprecursor aus der vorstehend genannten Gruppe auszuwählen ist. Vorzugsweise umfasst der Metallprecursor ein Metall, welches aus der Gruppe, bestehend aus einem Lanthanoid, wie beispielsweise La, Ce, Tb oder Eu, einem Übergangsmetall, wie beispielsweise Zn oder Mn, einem Erdalkalimetall, wie beispielsweise Ba, und Kombinationen davon, ausgewählt ist. Besonders bevorzugt ist der Metallprecursor aus der Gruppe, bestehend aus LaCl₃, CeCl₃, TbCl₃, EuCl₃, YCl₃, BaCl₂, ZnCl₂, MnCl₂, deren entsprechenden Kristallwasser-haltigen Verbindungen sowie Kombinationen davon, ausgewählt. In einer bevorzugten Ausführungsform der vorliegenden Erfindung werden mindestens zwei verschiedene Metallprecursor verwendet.

Das Fällungsreagens ist aus der Gruppe, bestehend aus Hydroxiden, Phosphaten, Carbonaten, Oxalaten, Sulfaten, Sulfiden, Halogeniden, Ammoniak, Hydroxylamin, Aminen, Amiden, lminen, Wasser und Kombinationen davon, ausgewählt. Dabei ist selbstverständlich, dass das Fällungsreagens von dem Metallprecursor verschieden ist und in geeigneter Weise auszuwählen ist, dass eine Ausfällung der gewünschten Leuchtstoffpartikelprecursor erreicht wird. Dabei ist dem Fachmann bekannt, in welcher Weise diese Auswahl stattzufinden hat. Vorzugsweise ist das Fällungsreagens aus der Gruppe, bestehend aus LiOH, NaOH, KOH, NH₃, [N(CH₃)₄]OH, H₃PO₄, (NH₄)H₂PO₄, NaHCO₃, NaCl, NaF, Na₂C₂O₄, H₂SO₄, Na₂SO₄, H₂S, (NH₂)₂CS; Na₂S, (NH₂)₂CO, Si(NH)₂, VO(OiPr)₃, Si(OC₂H₅)₄, H₂O und Kombinationen davon, ausgewählt.

Die Umsetzung des mindestens einen Metallprecursors mit dem mindestens einen Fällungsreagenz in Schritt (a) erfolgt in einem ersten Lösungsmittel oder Lösungsrnittelgemisch. Dieses erste Lösungsmittel oder Lösungsmittelgemisch kann jedes für diese Umsetzung geeignete Lösungsmittel umfassen. Vorzugsweise umfasst das erste Lösungmittel oder Lösungsmittelgemisch mindestens eine lonische Flüssigkeit ("ionic liquid", "IL"). Ionische Flüssigkeiten finden aufgrund ihrer besonderen Eigenschaften, unter anderem wegen ihrer hohen Siedepunkte, zunehmend Anwendung als Reaktionsmedium in der chemischen Synthese und sind im Stand der Technik, beispielsweise in P. Wasserscheid, T. Welton, lonic Liquids in Synthesis, Wiley-VCH, Weinheim, 2003**,** und M. Antonietti, D. Kuang, B. Smarsly, Y. Zhou, Angew. Chem. 2004, 116, 5096, ausführlich beschrieben-. Im erfindungsgemäßen Verfahren kann jede geeignete lonische Flüssigkeit als Lösungsmittel bzw. Lösungsmittelbestandteil verwendet werden.

Vorzugsweise ist die mindestens eine lonische Flüssigkeit aus Kationen und Anionen aufgebaut, wobei die Anionen aus der Gruppe, bestehend aus Chlorid, Bromid, lodid, Methansulfonat, Hydrogensulfat, Octylsulfat, Thiocyanat, p-Toluolsulfonat, Tetrafluoroborat, Hexafluorophosphat, Bis(pentaftuoroethyl)phosphinat, Bis[oxalato(2-)]-borat, Bis[1,2-benzoldiolato(2-)-O,O']borat, Trifluoracetat, Trifluorsulfonat, Dicyanamid, Tris(trifluormethylsulfonyl)methid, Bis(trifluormethylsulfonyl)imid, Tris(pentafluorethyl)trifluorphosphat, Ethylsulfat, Diethylphosphat, 2-(2-Methoxyethoxy)-ethylsulfat, Butylsulfat und Kombinationen davon, ausgewählt sind, und die Kationen, aus der Gruppe, bestehend aus monosubstituierten Imidazoliumderivaten, wie 1-Methyl-imidazolium, disubstituierten Imidazoliumderivaten, wie 1,3-Dimethylimidazolium, 1-Ethyl-3-methylimidazolium, 1-Butyl-3-methylimidazolium, 1-Propyl-3-methylimidazolium, 1-Hexyl-3-methyl-imidazolium, 3-Methyl-1-octylimidazolium, 1-Decyl-3-methylimidazolium, 1-Dodecyl-3-methylimidazolium, 3-Methyl-1-tetradecylimidazolium, 1-Hexadecyl-3-methylimidazolium, 1-Octadecyl-3-methylimidazolium, 1-Benzyl-3-methylimidazolium, 1-Phenylpropyl-3-methylimidazolium, trisubstituierten lmidazoliumderivaten, wie 1,2,3-Trimethylimidazolium, 1-Ethyl-2,3-dimethylimidazolium, 1-Butyl-2,3-dimethylimidazolium, 1-Propyl-2,3-dimethylimidazolium, 1-Hexyl-2,3-dimethylimidazolium, 1-Hexadecyl-2,3-dimethylimidazolium, Pyridiniumderivaten, wie *N*-Ethylpyridinium, N-Butylpyridinium, *N*-Butyl-3,4-dimethylpyridinium, *N*-Butyl-3,5-dimethylpyridinium, N-Butyl-3-methylpyridinium, *N*-Butyl-4-methylpyridinium, *N*-Hexylpyridinium, N-Octylpyridinium, 1-Ethyl-3-hydroxymethylpyridinium, Pyrrolidiniumderivaten, wie 1,1-Dimethylpyrrolidinium, 1-Ethyl-1-methylpyrrolidinium, 1,1-Dipropylpyrrolidinium, 1,1-Dibutylpyrrolidinium, 1-Butyl-1-methylpyrrolidinium, 1,1-Dihexylpyrrolidinium, 1-Hexyl-1-methylpyrrolidinium, 1-Methyl-1-octylpyrrolidinium, Phosphoniumderivaten, wie Tetrabutylphosphonium, Trihexyl(tetradecyl)phosphonium, Ammoniumderivaten, wie Tetramethylammonium, Tetraethylammonium, Tetrabutylammonium, Methyltrioctyl-ammonium, Ethyldimethylpropylammonium, Cyclohexyltrimethylammonium, Ethanolammonium, Guanidiniumderivaten, wie Guanidinium, *N,N,N',N'*-Tetramethyl-N"-ethylguanidinium, *N,N,N',N',N"*-Pentamethyl-*N"-*propylguanidinium, *N,N,N',N',N"*-Pentamethyl-*N"*-isopropylguanidinium, Hexamethylguanidinium, Isouroniumderivaten, wie . O-Methyl-*N,N,N',N'-*tetramethylisouronium, S-Ethyl-*N,N,N;N'*-tetramethylisothiouronium, Sulfoniumderivaten, wie Diethylmethethylsulfonium, und Kombinationen davon, ausgewählt sind. Vorzugsweise wird als lonische Flüssigkeit eine Flüssigkeit verwendet, welche als Kation ein quartäres Ammoniumion und als Anion ein Bis(trifluormethylsulfonyl)imidanion oder ein Trifluorsulfonylanion umfasst. Besonders bevorzugte lonische Flüssigkeiten stellen [MeBu₃N][(SO₂CF₃)₂N] und [MeBu₃N][CF₃SO₃] dar.

In einer bevorzugten Ausführungsform der vorliegenden Erfindung kann das erste Lösungsmittel oder Lösungsmittelgemisch neben mindestens einer lonischen Flüssigkeit weiter mindestens ein erstes Hilfslösungsmittel, ausgewählt aus der Gruppe, bestehend aus Aldehyden, Estern, Ethern, Thiolen, Aromaten, Alkanen, Halogenalkanen, Alkoholen, wie Methanol und Ethanol, Carbonsäureanhydriden, Aminen, Amiden, wie Dimethylformamid, Imiden, Ketonen, Carbon- und Sulfonsäuren, Sulfoxiden, wie Dimethylsulfoxid, flüssigem Ammoniak, flüssigem Schwefeldioxid, flüssigem Kohlendioxid und Kombinationen davon, umfassen. Besonders bevorzugt ist das erste Hilfslösungsmittel aus der Gruppe, bestehend aus Methanol, Ethanol, Dimethylformamid, Dimethylsulfoxid, flüssigem NH₃, flüssigem SO₂, flüssigem CO₂ und Kombinationen davon, ausgewählt.

Die Umsetzung des mindestens einen Metallprecursors mit dem mindestens einen Fällungsreagenz kann in jeder geeigneten Weise erfolgen. Beispielsweise kann die Umsetzung erfolgen, indem der mindestens eine Metallprecursor in mindestens einer lonischen Flüssigkeit und/oder mindestens einem Hilfslösungsmittel vorgelegt wird und anschließend das mindestens eine Fällungsreagens, gelöst in mindestens einer lonischen Flüssigkeit und/oder mindestens einem Hilfslösungsmittel, zugegeben wird. Es ist aber auch möglich, dass das mindestens eine Fällungsreagens in mindestens einer lonischen Flüssigkeit und/oder mindestens einem Hilfslösungsmittel vorgelegt wird und anschließend der mindestens eine Metallprecursor, gelöst in mindestens einer lonischen Flüssigkeit und/oder mindestens einem Hilfslösungsmittel, zugegeben wird. Weiterhin ist es möglich, dass die Zugabe der lonischen Flüssigkeit vollständig oder partiell gemeinsam mit dem Fällungsreagens oder erst nach beendeter Umsetzung des mindestens einen Metallprecursors mit dem mindestens einen Fällungsreagens erfolgt. In diesem Fall erfolgt die Umsetzung in dem ersten Lösungsmittel oder Lösungsmittelgemisch, welches im Wesentlichen mindestens eines der obengenannten Hilfslösungsmittel umfasst.

Die Umsetzung des mindestens einen Metallprecursors mit dem mindestens einen Fällungsreagenz erfolgt bei einer Temperatur im Bereich von -50°C bis 200°C, vorzugsweise in einem Bereich von 50°C bis 150°C. In einer besonders bevorzugten Ausführungsform der vorliegenden Erfindung erfolgt die Umsetzung bei einer Temperatur im Bereich von 60°C bis 80°C.

Im Schritt (a) des erfindungsgemäßen Verfahrens, d.h. bei der Umsetzung des mindestens einen Metallprecursors mit dem mindestens einen Fällungsreagenz, wird ein Leuchtstoffpartikel-Zwischenprodukt bzw. -Precursor gebildet. In diesem ersten Reaktionsschritt werden Morphologie und Partikelgröße der resultierenden Leuchtstoffpartikel bereits weitgehend festgelegt. Das erhaltene Leuchtstoffpartikel-Zwischenprodukt bleibt in der flüssigen Phase, d.h. in dem ersten Lösungsmittel oder Lösungsmittelgemisch, dispergiert.

In Schritt (b) des erfindungsgemäßen Verfahrens erfolgt anschließend das thermische Behandaln durch Mikrowelleneinstrahlung des in Schritt (a) erhaltenen Reaktionsgemisches bei einer Temperatur im Bereich von 150°C bis 700°C für eine Zeitdauer im Bereich von 5 Sekunden bis 30 Minuten in einem zweiten Lösungsmittel oder Lösungsmittelgemisch, welches mindestens eine lonische Flüssigkeit umfasst. Vorzugsweise erfolgt das thermische Behandeln durch Mikrowelleneinstrahlung bei einer Temperatur im Bereich von 200°C bis 700°C, mehr bevorzugt 150°C bis 500°C, noch bevorzugter in einem Bereich von 200°C bis 350°C.

Die dabei verwendete Ionische Flüssigkeit kann jede geeignete lonische Flüssigkeit sein. Vorzugsweise handelt es sich um mindestens eine der vorstehend bereits genannten lonischen Flüssigkeiten. Das in Schritt (b) verwendete zweite Lösungsmittel oder Lösungsmittelgemisch umfasst neben der mindestens einen lonischen Flüssigkeit vorzugsweise mindestens ein zweites Hilfslösungsmittel, ausgewählt aus der Gruppe, bestehend aus Aldehyden, Estern, Ethern, Thiolen, wie Dekanthiol, Aromaten, Alkanen, wie Dodekan, Halogenalkanen, Alkoholen, Polyolen, wie Glycerin und Diethylenglykol, Carbonsäureanhydriden, Aminen, wie Octylamin und Pyridin, Amiden, Imiden, Ketonen, Carbon- und Sulfonsäuren, wie Octansäure und Dodecylsulfat, Sulfoxiden, Phosphanen, wie Trioctylphosphan (TOP), Phosphanoxiden, wie Trioctylphosphanoxid (TOPO), Phosphaten, wie Octylphosphat, und Kombinationen davon. In einer weiteren Ausführungsform können Polymere, wie Polyacrylate, Polyamine, Polyurethane, Polyharnstoffe, Phenoplaste, Aminoplaste und Epoxidharze und Kombinationen davon sowie auch entsprechende Monomervorstufen der genannten Polymere und Kombinationen davon in der lonischen Flüssigkeit bzw. in dem zweiten Hilfslösungsmittel - gelöst sein oder als zweites Hilfslösungsmittel dienen.

Bei dem zweiten Hilfslösungsmittel handelt es sich vorzugsweise um ein hochsiedendes Lösungmittel. So weist das zweite Hilfslösungsmittel vorzugsweise einen Siedepunkt von mehr als 250°C, besonders bevorzugt von mehr als 300°C auf. Das zweite Hilfslösungsmittel kann beispielsweise direkt vor Schritt (b) des erfindungsgemäßen Verfahrens zu dem Reaktionsgemisch gegeben werden. Es ist aber auch möglich, dass das zweite Hilfslösungsmittel bereits in dem Lösungsmittel oder Lösungsmittelgemisch von Schritt (a) des erfindungsgemäßen Verfahrens enthalten ist.

In einer bevorzugten Ausführungsform der vorliegenden Erfindung werden Schritt (a) in einem ersten Lösungsmittel oder Lösungsmittelgemisch und Schritt (b) in einem zweiten Lösungsmittel oder Lösungsmittelgemisch durchgeführt, wobei beide Lösungsmittel oder Lösungsmittelgemische die gleiche lonische Flüssigkeit umfassen. Noch bevorzugter werden Schritt (a) in einem ersten Lösungsmittel oder Lösungsmittelgemisch und Schritt (b) in einem zweiten Lösungsmittel oder Lösungsmittelgemisch durchgeführt, wobei beide Lösungsmittel oder Lösungsmittelgemische die gleiche lonische Flüssigkeit und die gleichen Hilfslösungsmittel umfassen.

In der vorliegenden Erfindung erfolgt das thermische Behandeln für eine Zeitdauer von 5 Sekunden bis 30 Minuten, insbesondere 30 Sekunden bis 30 Minuten. Besonders bevorzugt ist eine Zeitdauer für die thermische Behandlung im Bereich von 5 Sekunden bis 600 Sekunden, noch bevorzugter im Bereich von 30 Sekunden bis 300 Sekunden. Eine kurze Dauer der thermischen Behandlung ist im Hinblick auf eine Verringerung der Agglomerationstendenz vorteilhaft.

Das thermische Behandeln erfolgt durch Mikrowelleneinstrahlung, da dieses ein kurzzeitiges Erwärmen auf hohe Temperaturen ermöglicht. Dabei ist eine Mikrowelleneinstrahlung mit 600 bis 1300 W über einen Zeitraum von 5 bis 600 Sekunden vorteilhaft, wobei das Reaktionsgemisch auf Temperaturen im Bereich von 150 bis 500°C erwärmt wird. Das gute Ansprechverhalten Ionischer Flüssigkeiten auf Mikrowelleneinstrahlung sowie deren hohe Siedepunkte gewährleisten einen effizienten Energieeintrag. Auf diese Weise ist vorteilhafterweise ein sehr schnelles (< 300 Sekunden) und direktes Erwärmen des Reaktionsgemisches bis hin zum Siedepunkt der lonischen Flüssigkeit möglich.

Das thermische Behandeln durch Mikrowelleneinstrahlung kann beispielsweise auch unter vermindertem Druck durchgeführt werden, um flüchtige Bestandteile, wie beispielsweise das erste Hilfslösungsmittel, aus dem Reaktionsgemisch zu entfernen. Das thermische Behandeln sowie das Entfernen flüchtiger Bestandteile kann gleichzeitig, aber auch in getrennten, nacheinander geschalteten Schritten erfolgen. In einer weiteren bevorzugten Ausführungsform umfasst das erfindungsgemäße Verfahren nach dem Schritt (a) des Umsetzens mindestens eines Metallprecursors mit mindestens einem Fällungsreagens weiter den Schritt des Entfernens niedrig siedender Hilfslösungsmittel. Sowohl der Schritt (b) des thermischen Behandelns als auch der Schritt des Entfernens flüchtiger Bestandteile vor, während oder nach Schritt (b) des thermischen Behandlens kann mehrfach erfolgen.

Im diesem zweiten Schritt wird die Kristallinität der geformten Leuchtstoffnanopartikel erhöht. Durch das thermische Behandeln werden Leuchtstoffpartikel erhalten, die einen Partikeldurchmesser von 1 bis 100 nm, eine nahezu monodisperse Größenverteilung im Bereich von ±20% und in Abhängigkeit von der gewählten Materialklasse eine Quantenausbeute von mindestens 20% aufweisen. Die so erhaltenen Leuchtstoffpartikel weisen eine hohe Kristallinität und einen geringen Agglomerationsgrad auf.

Da lonische Flüssigkeiten im Wesentlichen schwach-koordinierende Lösungsmittel sind, ist eine Stabilisierung von Partikeln mit Partikelgrößen im Bereich von 1 bis 100 nm, insbesondere gegen Agglomeration, bei hohen Temperaturen eigentlich nur eingeschränkt möglich. Dieses Problem wird erfindungsgemäß durch ein sehr direktes und vorzugsweise kurzzeitiges Erwärmen, beispielsweise durch Mikrowelleneinstrahlung, gelöst. Durch diese Art der thermischen Behandlung kann eine Agglomeration der Leuchtstoffpartikel mit Partikelgrößen im Bereich von 1 bis 100 nm wirksam vermieden werden. Die effiziente Unterdrückung der Agglomeratbildung ist - ohne daran gebunden zu sein - insofern wohl auf eine Ladungsstabilisierung zurückzuführen.

Nach erfolgter thermischer Behandlung folgt in Schritt (c) des erfindungsgemäßen Verfahrens das Isolieren und/oder Aufreinigen der Leuchtstoffpartikel. Dieses Isolieren und/oder Aufreinigen kann durch alle geeigneten Verfahren erfolgen. Derartige Verfahren sind im Stand der Technik bekannt.

Vorzugsweise erfolgt das Isolieren und/oder Aufreinigen der Leuchtstoffpartikel durch ein Verfahren, ausgewählt aus der Gruppe, bestehend aus Zentrifugationstechniken, Dialysetechniken, Phasentransfertechniken, Chromatographietechniken, Ultrafiltrationstechniken, Waschtechniken und Kombinationen davon. Beispielsweise kann das Isolieren und Aufreinigen erfolgen, indem das in Schritt (b) erhaltene Reaktionsgemisch bzw. die erhaltene Reaktionsdispersion mit einem der vorstehend beschriebenen Hilfslösungsmittel verdünnt und anschließend zentrifugiert wird. Auf diese Weise ist ein vollständiges Abtrennen des Reaktionsmediums, insbesondere der lonischen Flüssigkeit, im Gegensatz zu den im Stand der Technik verwendeten stark koordinierenden Lösungsmittel in einfacher Weise möglich.

Weiter können die erhaltenen Leuchtstoffpartikel zur Entfernung der lonischen Flüssigkeit auch mit geeigneten Waschlösungen gewaschen werden. Derartige Waschlösungen sind beispielsweise verdünnte Lösungen von Alkali-, Erdalkali-, Ammonium- oder Tetraalkylammoniumsalzen, wie Halogeniden, Carbonaten, Hydrogencarbonaten, Sulfaten, Hydrogerisulfaten, Acetaten, Oxalaten, Acetylacetonaten und Phosphaten, in Wasser, einem Alkohol, wie beispielsweise Methanol, Ethanol und Glycerin, oder einem Amin, wie beispielsweise Ethylendiamin, Ethylamin und Pyridin. Vorzugsweise wird zum Waschen eine 0,1 M NaCl-Lösung oder eine 0,1 M NH₄Cl-Lösung in Wasser oder Ethanol verwendet.

Die vorstehend genannten Verfahren zur Isolierung und/oder Aufreinigung der Leuchtstoffpartikel können auch kombiniert und/oder mehrfach ausgeführt wert den. Vorzugsweise beträgt der Restgehalt an lonischer Flüssigkeit in bzw. an den Leuchtstoffpartikeln weniger als 1 mol%, bevorzugter weniger als 10⁻³ mol%. Der Nachweis der auf den Oberflächen der Leuchtstoffpartikel verbliebenen Menge an Reaktionsmedium kann durch im Stand der Technik bekannte Verfahren erfolgen, beispielsweise durch Schwingungsspektroskopie, Massenspektrometrie, Kernresonanzspektroskopie, Chemische Analyse und Verbrennungsanalyse.

In einer weiteren bevorzugten Ausführungsform umfasst das erfindungsgemäße Verfahren vor oder nach dem Schritt (c) des lsolierens und/oder Aufreinigens der Leuchtstoffpartikel weiter einen Schritt des Beschichtens der Leuchtstoffpartikel mit einer anorganischen Hülle unter Bildung von Kern-Schale-Partikeln ("Core-Shell-Partikel"). Entsprechende Verfahren zur Herstellung von Kern-Schale-Partikeln sind im Stand der Technik bekannt. Das Material der Schale ist vorzugsweise aus der Gruppe, bestehend aus Phosphaten, Halogenphosphaten, Arsenaten, Sulfaten, Boraten, Aluminaten, Gallaten, Silicaten, Germanaten, Oxiden, Vanadaten, Niobaten, Tantalaten, Wolframaten, Molybdaten, Halogeniden und Alkalihalogeniden, Nitriden, Oxynitriden, Phosphiden, Sulfiden, Seleniden, Telluriden, Sulfoseleniden, Oxysulfiden und Kombinationen davon, ausgewählt. In einer bevorzugten Ausführungsform weist die Schale eine chemische Zusammensetzung auf, weiche der des Leuchtstoffpartikelkerns entspricht, jedoch nicht dotiert ist. Ebenfalls bevorzugt ist eine Schale, deren Zusammensetzung das Kation des Wirtsgitters des Leuchtstoffpartikelkerns und Fluorid oder Phosphat als Anion aufweist. In einer weiteren bevorzugten Ausführungsform ist die Schale aus einem Material, ausgewählt aus SiO₂, TiO₂, ZnO, Al₂O₃, ZrO₂, SnO₂, MgO, den Hydroxiden, Oxidhydroxiden, Oxidhydraten, Hydroxidhydraten der vorstehend genannten Oxide, MgF₂, CaF₂, SrF₂, BaF₂, ScF₃, YF₃, den Lanthanoidfluoriden und Kombinationen davon, aufgebaut.

Die vorliegende Erfindung betrifft ferner anorganische Leuchtstoffpartikel mit einem Partikeldurchmesser von 1 bis 100 nm, einer nahezu monodispersen Grö-βenverteilung im Bereich von ±20% und einer Quantenausbeute in Abhängigkeit von der gewählten Materialklasse von mindestens 20%, welche durch das erfindungsgemäße, vorstehend beschriebene Verfahren erhältlich sind. Vorzugsweise weisen die erfindungsgemäßen Leuchtstoffpartikel einen Partikeldurchmesser von 1 bis 20 nm auf. In Abhängigkeit von der gewählten Materialklasse können auch Quantenausbeuten von 40 % oder mehr bzw. 60 % oder mehr erhalten werden. Es ist weiter bevorzugt, dass die Leuchtstoffpartikel eine monodisperse Größenverteilung im Bereich von ±5% aufweisen. Im Stand der Technik sind geeignete Verfahren zur Bestimmung des Partikeldurchmessers, der monodispersen Größenverteilung sowie der Quantenausbeute bekannt.

Die gemäß dem erfindungsgemäßen Verfahren erhältlichen Leuchtstoffpartikel weisen eine hohe Kristallinität auf und können je nach Bedarf unterschiedlichste Anregungs- und Emissionscharakteristika aufweisen. Vorzugsweise weisen die erfindungsgemäßen Leuchtstoffpartikel eine Anregung im Bereich von 100 bis 800 nm und eine Emission im Bereich von 200 bis 2000 nm auf.

In einer bevorzugten Ausführungsform sind die erfindungsgemäßen Leuchtstoffpartikel mit 5 ppm bis 50 mol% eines Dotierungsmittel dotiert, wobei das Dotierungsmittel aus Elementen der Gruppe, bestehend aus Lanthanoiden, Übergangsmetallen, Hauptgruppenelementen und Kombinationen davon, ausgewählt ist. Besonders bevorzugt ist das Dotierungsmittel aus der Gruppe, bestehend aus La, Ce, Pr, Nd, Sm, Eu, Gd, Tb, Dy, Ho, Er, Tm, Yb, Lu, Cr, Mn, Cu, Zn, Y, Ag, Cd, B, Al, Ga, In, Ge, Sn, Pb, den Halogenen, den Chalkogenen, den Elementen der Stickstoffgruppe und Kombinationen davon, ausgewählt. Besonders bevorzugt ist eine Dotierung im Bereich von 0,1 bis 5,0 mol%.

Die gemäß dem erfindungsgemäßen Verfahren erhältlichen Leuchtstoffpartikel weisen vorzugsweise eine chemische Zusammensetzung, ausgewählt aus der Gruppe, bestehend aus Lil:Eu, Csl:Na, LiF:Mg, LiF:Mg,Ti, LiF:Mg,Na, KMgF₃:Mn, Al₂O₃:Eu, BaFCI:Eu, BaFCI:Sm, BaFBr:Eu, BaFCl_{0,5}Br_{0,5}:Sm, BaY₂F₈:A (A= Pr, Tm, Er, Ce), BaSi₂O₅:Pb, BaMg₂Al₁₆O₂₇:Eu, BaMgAl₁₃O₂₃:Eu, BaMgAl₁₀O₁₇:Eu, (Ba,Mg)Al₂O₄:Eu, Ba₂P₂O₇:Ti, (Ba,Zn,Mg)₃Si₂O₇:Pb, Ce(Mg,Ba)Al₁₀O₁₉, Ce_{0,60}Tb_{0,35}MgAl₁₀O₁₉, MgAl₁₁O₁₉:Ce,Tb, MgF₂:Mn, MgS:Eu, MgS:Ce, MgS:Sm, MgS:Sm,Ce, (Mg,Ca)S:Eu, MgSiO₃:Mn, 3,5MgOx0,5MgF₂xGeO₂:Mn, MgWO₄:Sm, MgWO₄:Pb, (Zn,Mg)F₂:Mn, (Zn,Be)SiO₄:Mn, Zn₂SiO₄:Mn, ZnO:Zn, ZnO:Zn,Si,Ga, Zn₃(PO₄)₂:Mn, ZnS:A (A = Ag, Al, Cu), (Zn,Cd)S:A (A = Cu, Al, Ag, Ni), CdBO₄:Mn, CaF₂:Mn, CaF₂:Dy, CaS:A (A = Lanthanoide, Bi), (Ca,Sr)S:Bi, CaWO₄:Pb, CaWO₄:Sm, CaSO₄:A (A = Mn, Lanthanoide), 3Ca₃(PO₄)₂xCa(F,Cl)₂₁Sb,Mn, CaSiO₃:Mn,Pb, Ca2Al₂Si₂O₇:Ce, (Ca,Mg)SiO₃:Ce, (Ca,M₉)SiO₃:Ti, 2Sr_{0,6}(B₂O₃)xSrF₂:Eu. 3Sr₃(PO₄)₂xCaCl₂:Eu, A₃(PO₄)₂xACl₂:Eu (A = Sr, Ca, Ba), (Sr,Mg)₂P₂O₇:Eu, (Sr,Mg)₃(PO₄)₂:Sn, SrS:Ce, SrS:Sm,Ce, SrS:Sm, SrS:Eu, SrS:Eu,Sm, SrS:Cu,Ag, Sr₂P₂O₇:Sn, Sr₂P₂O₇:Eu, Sr₄Al₁₄O₂₅:Eu, SrGa₂S₄:A (A = Lanthanoide, Pb), SrGa₂S₄:Pb, Sr₃Gd₂Si₆0₁₈:Pb,Mn, YF₃:Yb,Er, YF₃:Ln (Ln = Lanthanoide), YLiF₄:Ln (Ln = Lanthanoide), Y₃Al₅O₁₂:Ln (Ln = Lanthanoide), YAl₃(BO₄)₃:Nd,Yb, (Y,Ga)BO₃:Eu, (Y,Gd)BO₃:Eu, Y₂Al₃Ga₂O₁₂:Tb, Y₂SiO₅:Ln (Ln = Lanthanoide), Y₂O₃:Ln (Ln = Lanthanoide), Y₂O₂S:Ln (Ln = Lanthanoide), YVO₄:A (A = Lanthanoide, In), Y(P,V)O₄:Eu, YTaO₄:Nb, YAlO₃:A (A = Pr, Tm, Er, Ce), YOCI:Yb,Er, LnPO₄:Ce,Tb (Ln = Lanthanoide oder Gemische von Lanthanoiden), LuVO₄:Eu, GdVO₄:Eu, Gd₂O₂S:Tb, GdMgB₅O₁₀:Ce,Tb, LaOBr:Tb, La₂O₂S:Tb, NaGdF₄:Yb,Er, NaLaF₄:Yb,Er, LaF₃:Yb,Er,Tm, BaYF₅:Yb,Er, Ga₂O₃:Dy, GaN:A (A = Pr, Eu, Er, Tm), Bi₄Ge₃O₁₂, LiNbO₃:Nd,Yb, LiNbO₃:Er, LiCaAlF₆:Ce, LiSrAlF₆:Ce, LiLuF₄:A (A = Pr, Tm, Er, Ce), Gd₃Ga₅O₁₂:Tb, Gd₃Ga₅O₁₂:Eu, Li₂B₄O₇:Mn,SiOₓ,Er,Al (0<x<2), YVO₄:Eu, YVO₄:SM, YVO₄:Dy, LaPO₄:Eu, La-PO₄:Ce, LaPO₄:Ce,Tb, ZnS:Tb, ZnS:TbF₃, ZnS:Eu, ZnS:EuF₃, Y₂O₃:Eu, Y₂O₂S:Eu, Y₂SiO₅:Eu, SiO₂:Dy, SiO₂:Al, Y₂O₃:Tb, ZnS:Tb, ZnS:Ag, ZnS:Cu, Ca₃(PO₄)₂:Eu, Ca₃(PO₄)₂:Eu,Mn, Sr₂SiO₄:Eu, Ba₂SiO₄:Eu, BaAl₂O₄:Eu, MgF₂:Mn, ZnS:Mn, ZnS:Ag, ZnS:Cu, CaSiO₃:A, CaS:A, CaO:A, ZnS:A, Y₂O₃:A, MgF₂:A (A = Lanthanoide), MS, MSe, MTe (M = Zn, Cd, Ge, Sn, Pb), MN, MP, MAs, MSb (M = Al, Ga, In), M₂SiO₄:Eu (M = Ca, Sr, Ba), M₂Si₅N₈:Eu (M = Ca, Sr, Ba), LaSi₃N₅:Ce, Ln₁₋ₓSrₓSi₃₋₂,Al₂ₓO₃ₓN₅₋₃ₓ:Ce, LaSi₃N₅:Ce und Ln₁₋ₓSrₓSi₃₋₂ₓAl₂ₓO₃ₓN₅₋₃ₓ:Eu (Ln = Al, Y, La, Lanthanoid) und Kombinationen davon, auf.

Das erfindungsgemäße Verfahren zur Herstellung von Leuchtstoffpartikeln sowie die gemäß diesem Verfahren erhältlichen Leuchtstoffpartikel weisen im Vergleich zum Stand der Technik wesentliche Vorteile auf. So kann durch das thermische Behandeln durch Mikrowelleneinstrahlung eine hohe Kristallinität der Leuchtstoffpartikel erreicht werden, was eine hohe Quanteneffizienz bedingt. Ferner ermöglicht das erfindungsgemäße Verfahren einen breiten synthetischen Zugang zu Leuchtstoffpartikeln mit Partikelgrößen im Bereich von 1 bis 100 nm mit unterschiedlichsten chemischen Zusammensetzungen sowie unterschiedlichsten Anregungs- und Emissionseigenschaften. Außerdem weisen die so erhaltenen Leuchtstoffpartikel eine hohe chemische Reinheit, eine nahezu monodisperse Größenverteilung sowie eine geringe Agglomerationstendenz auf.

Ferner ist aufgrund der geringen Oberflächenanhaftung des Reaktionsmediums auf den Partikeloberflächen eine vollständige Entfernung des Reaktionsmediums von den Partikeloberflächen möglich. Aufgrund der geringen Oberflächenanhaftung des Reaktionsmediums ist insofern auch ein einfacher Austausch gegen stärker anhaftende und stärker oberflächenaktive Substanzen und Stabilisatoren möglich. Die erfindungsgemäß erhältlichen Leuchtstoffpartikel weisen somit in besonders vorteilhafter Weise eine Oberflächenausstattung auf, welche ein agglomeratfreies Redispergieren in polaren sowie unpolaren Lösungs- bzw. Dispersionsmitteln ermöglicht. Durch die Wahl des Dispersionsmittels können die erfindungsgemäß erhältlichen Leuchtstoffpartikel in ihren chemisch-physikalischen Eigenschaften speziell an die Anforderungen einer bestimmten Anwendung angepasst werden.

Weitere vorteilhafte Aspekte des erfindungsgemäßen Verfahrens liegen in der Vermeidung toxischer Feststoffe, kurzen Reaktionszeiten sowie einer einfachen und schnellen Durchführbarkeit der Synthese. Außerdem ist es möglich, die im erfindungsgemäßen Verfahren verwendeten lonischen Flüssigkeiten und Hilfslösungsmittel wiederzuverwenden, was aus Umweltsgesichtspunkten vorteilhaft ist. Das erfindungsgemäße Verfahren kann ferner in der Form eines kontinuierliches Prozesses geführt werden, was für grosstechnische Anwendungen einen Vorteil darstellt.

Wie bereits erwähnt, liegt ein besonderer Vorteil der erfindungsgemäßen Leuchtstoffpartikel darin, dass infolge der geringen Oberflächenanhaftung des Reaktionsmediums die Oberflächenausstattung der erfindungsgemäß erhältlichen Partikel im Gegensatz zum Stand der Technik frei gewählt werden kann. Nach Austausch des Reaktionsmediums können dabei durch Zugabe geeigneter stärker anhaftender Stabilisatoren die Partikel eine Oberflächenausstattung aufweisen, welche ein agglomeratfreies Redispergieren in polaren sowie unpolaren Lösungsmitteln ermöglicht. Eine besondere Ausführungsform ist dabei das Redispergieren der erfindungsgemäßen Leuchtstoffpartikel in Gegenwart von Biomolekülen. Dieses ist insbesondere für biologisch-medizinische Anwendungen der erfindungsgemäßen Leuchtstoffpartikel vorteilhaft. Geeignete Biomoleküle sind beispielsweise Aminosäuren, Kohlenhydrate, Antikörper, Proteine, Peptide, Nucleoside, Nucleotide, Nucleinsäuren, DNA, DNA-Fragmente, Lipide, Terpene wie Steroide, Alkaloide und Kombinationen davon.

Die vorliegende Erfindung betrifft ferner die Verwendung der erfindungsgemäßen Leuchtstoffpartikel zur Beschichtung auf oder zur Einbettung in ein Substrat, wobei das Substrat aus der Gruppe, bestehend aus einem Papier-, Polymer-, Glas-, Metall- oder Keramiksubstrat, ausgewählt ist, beispielsweise zur Diagnostik, Therapie, als Kontrastmittel, als Markierung, Sicherheitsmerkmal, zur Beleuchtung, Datenspeicherung und/oder Datenvervielfältigung.

Besonders bevorzugt ist die Einbettung der Leuchtstoffpartikel in der Form einer transparenten und lumineszierenden Schicht oder Folie. Derartige lumineszierende Schichten oder Folien können beispielsweise als Markierung, Sicherheitsmerkmal, zur Beleuchtung, Datenspeicherung oder Datenvervielfältigung verwendet werden.

Für eine medizinisch-pharmazeutisch-biologische Verwendung der erfindungsgemäßen Leuchtstoffpartikel können diese beispielsweise in der Form von transparenten, lumineszierenden und biokompatiblen Dispersionen oder Pulver verwendet werden. Gerade für derartige Anwendungen ist das erfindungsgemäße Herstellungsverfahren vorteilhaft, da stark toxische Komponenten während der Synthese vermieden werden können. Derartige lumineszierende Dispersionen oder Pulver können beispielsweise zur Tumordiagnostik, Tumortherapie, zur Markierung von Plaques, zur Zellmarkierung, in FRET-Assays oder in Immuno-Assays verwendet werden.

Die vorliegende Erfindung betrifft ferner ein Verfahren zum Hersteller eines beschichteten Substrates, umfassend die Schritte (a) des Redispergierens der erfindungsmäß erhältlichen Leuchtstoffpartikel in einem Dispersionsmedium, (b) des Aufbringens der Dispersion auf ein, vorzugsweise transparentes, Substrat, und (c) des Trocknens der auf das Substrat aufgebrachten Dispersion oder das Zulassen, dass die auf das Substrat aufgebrachte Dispersion trocknet. Dabei erfolgt die Aufbringung der Leuchtstoffpartikel auf das Substrat vorzugsweise in der Form einer transparenten und lumineszierenden Beschichtung.

Das Dispersionsmedium kann jedes geeignete Dispersionsmedium sein. Vorzugsweise ist das Dispersionsmedium aus der Gruppe, bestehend aus Aldehyden, Estern, Ethern, Thiolen, wie Dekanthiol, Aromaten, Alkanen, wie Dodekan, Halogenalkanen, Alkoholen, wie Methanol und Ethanol, Polyolen, wie Glycerin und Diethylenglykol, Carbonsäureanhydriden, Aminen, wie Octylamin und Pyridin, Amiden, wie Dimethylformamid, Imiden, Ketonen, Carbon- und Sulfonsäuren, wie Octansäure und Dodecylsulfat, Sulfoxiden, wie Dimethylsulfoxid, Phosphanen, wie Trioctylphosphan (TOP), Phosphanoxiden, wie Trioctylphosphanoxid (TOPO), Phosphaten, wie Octylphosphat, Polymeren, wie Polyacrylaten, Polyaminen, Polyurethanen, Polyharnstoffen, Phenoplasten, Aminoplasten und Epoxidharzen, und Kombinationen davon, flüssigem Ammoniak, flüssigem Schwefeldioxid, flüssigem Kohlendioxid, Lösungen eines Alkali-, Erdalkali-, Ammonium- und Tetramethylammoniumsalzes, wie Halogeniden, Carbonaten, Hydrogencarbonaten, Sulfaten, Hydrogensulfaten, Nitraten, Acetaten, Oxalaten, Acetylacetonaten, Phosphaten in Wasser, einem Alkohol, wie Methanol, Ethanol und Glycerin, oder einem Amin, wie Ethylendiamin, Ethylamin und Pyridin, und Kombinationen davon, ausgewählt. In einer weiteren Ausführungsform können Polymere, wie Polyacrylate, Poly-amine, Polyurethane, Polyharnstoffe, Phenoplaste, Aminoplaste und Epoxidharze und Kombinationen davon sowie auch entsprechende Monomervorstufen der genannten Polymere und Kombinationen davon als Dispersionsmedium gewählt werden.

Die vorliegende Erfindung wird durch die nachstehenden, nicht-beschränkenden Beispiele weiter erläutert.

### Beispiele

### Beispiel 1:

In einem Schlenk-Gefäß werden 124 mg (0,334 mmol) LaCl₃ x 7 H₂O, 140 mg (0,376 mmol) CeCl₃ x 7 H₂O und 47 mg (0,126 mmol) TbCl₃ x 6 H₂O in einem Gemisch aus 5 ml Ethanol und 10 ml [MeBu₃N][(SO₂CF₃)₂N] gelöst. In einem weiteren Schlenk-Gefäß werden 163 mg (1,66 mmol) H₃PO₄ in einem Gemisch aus 2 ml Ethanol und 10 ml [MeBu₃N][(SO₂CF₃)₂N] gelöst. Anschließend wird unter Rühren die Lösung der Lanthanoid-Salze zu der H₃PO₄-Lösung bei einer Temperatur von 70°C zugetropft. Ethanol und überschüssige Phosphorsäure werden unter vermindertem Druck entfernt, wobei die Temperatur zunächst auf 100°C und dann bei nachlassender Gasentwicklung auf 150°C erhöht wird. Anschließend wird das Reaktionsgemisch unter Mikrowelleneinstrahlung auf 300°C erhitzt. Man lässt auf Raumtemperatur abkühlen, verdünnt mit 20 ml Ethanol und isoliert die Partikel durch Zentrifugation. LaPO₄:Ce,Tb wird mit einem Partikeldurchmesser von etwa 15 nm und einer Größenverteilung von ±20% erhalten. Als Quantenausbeute wird ein Wert von 65% erhalten.

### Beispiel 2:

In einem Schlenk-Gefäß werden 124 mg (0,334 mmol) LaCl₃ x 7 H₂O, 140 mg (0,376 mmol) CeCl₃ x 7 H₂O und 47 mg (0,126 mmol) TbCl₃ x 6 H₂O in einem Gemisch aus 5 ml Ethanol und 10 ml [MeBu₃N][CF₃SO₃] gelöst. In einem weiteren Schlenk-Gefäß werden 163 mg (1,66 mmol) H₃PO₄ in einem Gemisch aus 2 ml Ethanol und 10 ml [MeBu₃N][CF₃SO_{3]} gelöst. Anschließend wird unter Rühren die Lösung der Lanthanoid-Salze zu der H₃PO₄-Lösung bei einer Temperatur von 70°C zugetropft. Ethanol und überschüssige Phosphorsäure werden unter vermindertem Druck entfernt, wobei die Temperatur zunächst auf 100°C und dann bei nachlassender Gasentwicklung auf 150°C erhöht wird. Anschließend wird das Reaktionsgemisch unter Mikrowelleneinstrahlung auf 300°C erhitzt. Man lässt auf Raumtemperatur abkühlen, verdünnt mit 20 ml Ethanol und isoliert die Partikel durch Zentrifugation. LaP0₄:Ce,Tb wird mit einem Partikeldurchmesser von etwa 15 nm und einer Größenverteilung von ±20% erhalten. Als Quantenausbeute wird ein Wert von 65% erhalten.

### Beispiel 3:

In einem Schlenk-Gefäß werden 176 mg (0,474 mmol) LaCl₃ x 7 H₂O und 9 mg (0,025mmol) EuCl₃ x 6 H₂O in einem Gemisch aus 5 ml Ethanol und 10 ml [MeBu₃N][CF₃SO₃] gelöst. In einem weiteren Schlenk-Gefäß werden 100 mg (1,02 mmol) H₃PO₄ in einem Gemisch aus 2 ml Ethanol und 10 ml [MeBu₃N][CF₃SO₃] gelöst. Anschließend wird unter Rühren die Lösung der Lanthanoid-Salze zu der H₃PO₄-Lösung bei einer Temperatur von 70°C zugetropft. Ethanol und überschüssige Phosphorsäure werden unter vermindertem Druck entfernt, wobei die Temperatur zunächst auf 100°C und dann bei nachlassender Gasentwicklung auf 150°C erhöht wird. Anschließend wird das Reaktionsgemisch unter Mikrowelleneinstrahlung auf 300°C erhitzt. Man lässt auf Raumtemperatur abkühlen, verdünnt mit 20 ml Ethanol und isoliert die Partikel durch Zentrifugation. LaPO₄:Eu wird mit einem Partikeldurchmesser von etwa 10 nm und einer Größenverteilung von ±20% erhalten. Als Quantenausbeute wird ein Wert von 25% erhalten.

### Beispiel 4:

In einem Schlenk-Gefäß werden 151 mg (0,5 mmol) YCl₃ x 6 H₂O und 19 mg (0,053 mmol) EuCl₃ x 6 H₂O in einem Gemisch aus 5 ml Ethanol und 10 ml [MeBu₃N][CF₃SO₃] gelöst. In einem weiteren Schlenk-Gefäß werden 122 mg (0,5 mmol) VO(O-iPr)₃ in einem Gemisch aus 2 ml Ethanol und 10 ml [MeBu₃N][CF₃SO₃] gelöst. Anschließend werden die Lösungen bei einer Temperatur von 70°C zusammengegeben und für 2 Stunden gerührt. Ethanol wird unter vermindertem Druck entfernt, wobei die Temperatur zunächst auf 100°C und dann bei nachlassender Gasentwicklung auf 150°C erhöht wird. Anschließend wird das Reaktionsgemisch unter Mikrowelleneinstrahlung auf 300°C erhitzt. Man lässt auf Raumtemperatur abkühlen, verdünnt mit 20 ml Ethanol und isoliert die Partikel durch Zentrifugation. YVO₄:Eu wird mit einem Partikeldurchmesser von etwa 30 nm und einer Größenverteilung von ±10% erhalten. Als Quantenausbeute wird ein Wert von über 50% erhalten.

### Beispiel 5:

In einem Schlenk-Gefäß werden 159 mg (0,65 mmol) BaCl₂ x 2 H₂O und 7 mg (0,02 mmol) EuCl₃ x 6 H₂O in einem Gemisch aus 5 ml Ethanol und 10 ml [MeBu₃N][CF₃SO₃] gelöst. In einem weiteren Schlenk-Gefäß werden 68 mg (0,326 mmol) Si(OC₂H₅)₄ in einem Gemisch aus 2 ml Ethanol und 10 ml [MeBu₃N][CF₃SO₃] gelöst. Anschließend werden die Lösungen bei einer Temperatur von 70°C zusammengegeben. Es wird 1 mg (0,005 mmol) Me₄NOH x 5 H₂O, gelöst in 0,5 ml Ethanol, zugefügt und das Reaktionsgemisch für 4 Stunden gerührt. Ethanol wird unter vermindertem Druck entfernt, wobei die Temperatur zunächst auf 100°C und dann bei nachlassender Gasentwicklung auf 150°C erhöht wird. Anschließend wird das Reaktionsgemisch unter Mikrowelleneinstrahlung auf 300°C erhitzt. Man lässt auf Raumtemperatur abkühlen, verdünnt mit 20 ml Ethanol und isoliert die Partikel durch Zentrifugation. Ba₂SiO₄:Eu wird mit einem Partikeldurchmesser von etwa 20 nm und einer Größenverteilung von ±20% erhalten. Als Quantenausbeute wird ein Wert von 40% erhalten.

### Beispiel 6:

In einem Schlenk-Gefäß werden 87 mg (0,64 mmol) ZnCl₂ und 8 mg (0,04 mmol) MnCl₂ x 4 H₂O in einem Gemisch aus 5 ml Ethanol und 10 ml [MeBu₃N][CF₃SO₃] gelöst. In einem weiteren Schlenk-Gefäß werden 66 mg (0,32 mmol) Si(OC₂H₅)₄ in einem Gemisch aus 2 ml Ethanol und 10 ml [MeBu₃N][CF₃SO₃] gelöst. Anschließend werden die Lösungen bei einer Temperatur von 70°C zusammengegeben. Es wird 1 mg (0,005 mmol) Me₄NOH x 5 H₂O, gelöst in 0.5 ml Ethanol, zugefügt und das Reaktionsgemisch für 4 Stunden gerührt. Ethanol wird unter vermindertem Druck entfernt, wobei die Temperatur zunächst auf 100°C und dann bei nachlassender Gasentwicklung auf 150°C erhöht wird. Anschließend wird das Reaktionsgemisch unter Mikrowelleneinstrahlung auf 300°C erhitzt. Man lässt auf Raumtemperatur abkühlen, verdünnt mit 20 ml Ethanol und isoliert die Partikel durch Zentrifugation. Zn₂SiO₄:Mn wird mit einem Partikeldurchmesser von etwa 10 nm und einer Größenverteilung von ±10% erhalten. Als Quantenausbeute wird ein Wert von 35% erhalten.

## Patentansprüche

1. Verfahren zur gezielten Synthese von Leuchtstoffpartikeln, welche einen Partikeldurchmesser von 1 bis 100 nm, eine nahezu monodisperse Größenverteilung im Bereich von ±20% und in Abhängigkeit von der gewählten Materialklasse eine Quantenausbeute von mindestens 20% aufweisen, umfassend die Schritte:
(a) das Umsetzen mindestens eines Metallprecursors mit mindestens einem Fällungsreagens in einem ersten Lösungsmittel oder Lösungsmittelgemisch bei einer Temperatur im Bereich von -50°C bis 200°C unten, Erhalten eines ersten Reaktionsgemisches,
(b) das thermische Behandeln des in Schritt (a) erhaltenen ersten Reaktionsgemisches bei einer Temperatur im Bereich von 150°C bis 700°C für eine Zeitdauer im Bereich von 5 Sekunden bis 30 Minuten in einem zweiten Lösungsmittel oder Lösun2,sgittelgemisch, welches mindestens eine lonische Flüssigkeit umfasst, wobei das thermische Behandeln durch Mikrowelleneinstrahlung erfolgt und
(c) das Isolieren und/oder Aufreinigen der Leuchtstoffpartikel,
wobei der mindestens eine Metallprecursor aus der Gruppe, bestehend aus Metallhalogeniden, -nitraten, -sulfaten, -hydrogensulfaten, -acetaten, -acetytacetonaten. -oxalaten, -carbonaten, -hydrogencarbonaten, -phosphaten, -hydrogenphosphateh, -dihydrogenphosphaten, -hydriden, -alkoholaten, metallorganischen Verbindungen und Kombinationen davon ausgewählt ist, und
wobei das Fällungsreagens aus der Gruppe, bestehend aus Hydroxiden, Phosphaten, Carbönaten, Oxalaten, Sulfaten, Sulfiden, Halogeniden, Ammoniak, Hydroxylamin, Aminen, Amiden, Iminen, Wasser und Kombinationen davon, ausgewählt ist.

2. Verfahren nach Anspruch 1, wobei die mindestens eine Ionische Flüssigkeit aus Kationen und Anionen aufgebaut ist, wobei die Anionen aus der Gruppe, bestehend aus Chlorid, Bromid, lodid, Methansulfonat, Hydrogensulfat, Octylsulfat, Thiocyanat, p-Toluolsulfonat, Tetrafluoroborat, Hexafluorophosphat, Bis(pentafluoroethyl)phosphinat, Bis[oxalato(2-)]-borat, Bis[1,2-benzoldiolato(2-)-O,O]brat, Trifluoracetat, Trifluorsulfonat, Dicyanamid, Tris(trfflüormethylsulfonyl)methid, Bis(trifluormethylsuffonyl)imid, Tris(pentafluorethyl)trifluorphosphat, Ethylsulfat, Diethylphosphat, 2-(2-Methoxyethoxy)ethysulfat, Butylsulfat und Kombinationen davon, ausgewählt sind, und die Kationen, aus der Gruppe, bestehend aus monosubstituierten Imidazoliumderivaten, wie 1-Methylimidazolium, disubstituierten Imidazoliumderivaten, wie 1,3-Dimethylimidazolium, 1-Ethyl-3-methylimidazolium, 1-Butyl-3-methylimidazolium, 1-Propyl-3-methylimidazolium, 1-Hexyl-3-methylimidazolium, 3-Methyl-1-octylimidazolium; 1-Decyl-3-methylimidazolium, 1-Dodecyl-3-methylimidazolium, 3-Methyl-1-tetradecylimidazolium, 1-Hexadecyl-3-methylimidazolium, 1-Odadecyl-3-methylimidazolium, 1-Benzyl-3-methylimidazolium, 1-Phenylpropyl-3-methylimidazolium, trisubstituierten Imidazoliumderivaten, wie 1,2,3-Trimethylimidazoliüm, 1-Ethyl-2,3-dimethylimidazolium, 1-Butyl-2,3-dimethylimidazolium, 1-Propyl-2,3-dimethylimidazolium, 1-Hexyl-2,3-dirriethylimidazollum, 1-Hexadecyl-2,3-dimethylimidazolurm, Pyridiniumderivaten, wie *N*-Ethylpyridinium, *N-*Butylpyridinium, *N*-Butyl-3,4-dimethylpyridinium, *N*-Butyl-3,5-dimethytpyndinium, *N*-Butyl-3-methylpyridinium, *N*-Butyl-4-methylpyridinium, *N*-Hexylpyridinium, *N*-Octylpyridinium, 1-Ethyl-3-hydroxymethylpyridinium, Pyrrolidiniumderivaten, wie 1,1-Dimethylpyrrolidinium, 1-Ethyl-1 methylpymolidinium, 1,1-Dipropylpyrrolidinium, 1,1-Dibutylpyrrolidinium, 1-Butyl-1-methylpymolidinium, 1,1-Dihexylpyrrolidinium, 1-Hexyl-1-methylpyrrolidinium, 1-Methyl-1-octylpyrrolidinium, Phosphoniumderivaten, wie Tetrabutylphosphonium, Trihexyl(tetradecyl)phosphonurm, Ammoniumderivaten, wie Tetramethylammonium, Tetraethylammonium, Tetrabutylammonium, Methyltrioctylammonium, Ethyldimethylpropylammonium; Cyclohexyltrimethylammonium, Ethanolammonium, Guanidiniumderivaten, wie Guanidinium, *N,N,N',N'*-Tetramethyl-*N*"-ethylguanidinium, *N,N,N',N',N"-*Pentamethyl-*N"*-propylguanidinium, *N,N,N;N;N"*-Pentamethyl-*N"-*isopropylguanidinium, Hexamethylguanidinium, Isouroniumderivaten, wie *O-*Methyl-*N,N,N',N'*-tetramethylisouronium, S-Ethyl-*N,N,N',N.'-*tetramethytisothiouronium, Sulfoniumderivaten, wie Diethylmethethylsulfonium, und Kombinationen davon, ausgewählt sind.

3. Verfahren nach Anspruch 1 oder 2, wobei das in Schritt (a) verwendete erste Lösungsmittel oder Lösungsmittelgemisch weiter mindestens ein erstes Hilfslösungsmittel, ausgewählt aus der Gruppe, bestehend aus Aldehyden Estern, Ethern, Thiolen, Aromaten, Alkanen, Halogenalkanen, Alkoholen, wie Methanol und Ethanol, Carbonsäureanhydriden, Aminen. Amiden, wie Dimethylformamid, imiden. Ketonen, Carbon- und Sulfonsäuren, Sulfoxiden, wie Dimethylsulfoxid, flüssigem Ammoniak, flüssigem Schwefeldioxid, flüssigem Kohlendioxid und Kombinationen davon, umfasst.

4. Verfahren nach Anspruch 3, welches nach dem Schritt (a) des Umsetzens mindestens eines Metallprecursors mit mindestens einem Fällungsreagens weiter den Schritt des Entfernens niedrig siedender Hilfslösungsmittel umfasst.

5. Verfahren nach einem der Ansprüche 1 bis 4, wobei das in Schritt (b) verwendete zweites Lösungsmittel oder Lösungsmittelgemisch mindestens ein zweites Hilfslöäungsmittel, ausgewählt aus der Gruppe, bestehend aus Aldehyden, Estern, Ethern, Thiolen, wie Dekanthiol, Aromaten, Alkanen, wie Dodekan, Halogenalkanen, Alkoholen, Polyolen, wie Glycerin und Diethyl lenglykol, Carbonsäureanhydriden, Aminen, wie Octylamin und Pyridin, Amiden, Imiden, Ketonen, Carbon- und Suffonsäuren, wie Octansäure und Dodecylsulfat, Sulfoxiden, Phosphanen, wie Trioctylphosphan (TOP), Phosphanoxiden, wie Trioctylphosphanoxid (TOPO), und Phosphaten, wie Octylphosphat, umfasst.

6. Verfahren nach einem der Ansprüche 1 bis 5, wobei das thermische Behandeln in Schritt (b) für eine Zeitdauer von 30 Sekunden bis 600 Sekunden erfolgt.

7. Verfahren nach einem der Ansprüche 1 bis 6, wobei das Isolieren und/oder Aufreinigen der Leuchtstoffpartikel durch ein Verfahren, ausgewählt aus der Gruppe, bestehend aus Zentrifugationstechniken, Dialysetechniken, Phasentransfertechniken, Chromatographietechniken, Uftrafiltrationstechniken, Waschtechniken und Kombinationen davon, erfolgt.

8. Verfahren nach einem der Ansprüche 1 bis 7, welches vor oder nach dem Schritt (c) des Isolierens und/oder Aufreinigens der Leuchtstoffpartikel weiter einen Schritt des Beschichtens der Leuchtstoffpartikel mit einer anorganischen Hülle unter Bildung von Kem-Schale-Partikeln umfasst.

9. Anorganische Leuchtstoffpartikel mit einem Partikeldurchmesser von 1 bis 100 nm, einer nahezu monodispersen Größenverteilung im Bereich von ±20% und einer Quantenausbeute von mindestens 20%, erhältlich durch das Verfahren nach einem der Ansprüche 1 bis 8.

10. Anorganische Leuchtstoffpartikel nach Anspruch 9, welche mit 5 ppm bis 50 mol% eines Dotierungsmittel dotiert sind, wobei das Dotierungsmittel aus der Gruppe, bestehend aus Lanthanoiden, Übergangsmetallen, Hauptgruppenelementen und Kombinationen davon, ausgewählt ist.

11. Anorganische Leuchtstonpartikel nach Anspruch 9 oder 10, welche eine chemische Zusammensetzung, ausgewählt aus der Gruppe, bestehend aus Lil:Eu, Csl:Na, LiF:Mg, LiF:Mg,Ti, LiF:Mg,Na, KMgF₃:Mn, Al₂O₃:Eu, BaFCl:Eu, BaFCl:Sm, BaFBr.Eu, BaFCl_{0,5}Br_{0,5}:Sm, BaY₂F₈:A (A= Pr, Tm, Er, Ce), BaSi₂O₅:Pb, BaMg₂Al₁₆O₂₇:Eu, BaMgAl₁₃O₂₃:Eu, BaMgAl₁₀O₁₇:Eu, (Ba,Mg)Al₂O₄:Eu, Ba₂P₂O₇:Ti, (Ba,Zn,Mg)₃Si₂O₇:Pb, Ce(Mg,Ba)Al₁₁O₁₉, Ce_{0,60}Tb_{0,35}MgAl₁₀O₁₉, MgAl₁₁O₁₉:Ce,Tb, MgF₂:Mn, MgS:Eu, MgS:Ce, MgS:Sm, MgS:Sm,Ce, (Mg,Ca)S:Eu, MgSiO₃:Mn, 3,5MgOx0,5MgF₂xGeO₂:Mn, MgWO₄:Sm, MgWO₄:Pb, (Zn,Mg)F₂:Mn, (Zn,Be)SiO₄:Mn, Zn₂SiO₄:Mn, ZnO:Zn, ZnO:Zn,Si,Ga, Zn₃(PO₄)₂:Mn, ZnS:A (A = Ag, Al, Cu), (Zn,Cd)S:A (A= Cu, Al, Ag, Ni), CdBO₄:Mn, CaF₂:Mn, CaF₂:Dy, CaS:A (A = Lanthanoide, Bi), (Ca,Sr)S:Bi, CaWO₄:Pb, CaWO₄:Sm, CaSO₄:A (A = Mn, Lanthanoide), 3Ca₃(PO₄)₂xCa(F,Cl)₂:Sb,Mn, CaSiO₃:Mn,Pb, Cₐ₂Al₂Si₂O₇:Ce, (Ca,Mg)SiO₃:Ce, (Ca,Mg)SiO₃:Ti, 2Sr_{0,6}(B₂0₃)xSrF₂:Eu, 3Sr₃(PO₄)₂xCaCl₂:Eu, A₃(PO₄xACl₂:Eu (A =Sr, Ca, Ba), (Sr,Mg)₂P₂O₇:Eu, (Sr,Mg)₃(PO₄)₂:Sn, SrS:Ce, SrS:Sm,Ce, SrS:Sm, SrS:Eu, SrS:Eu,Sm, SrS:Cu,Ag, Sr₂P₂O₇:Sn, Sr₂P₂₀O₇:Eu, Sr₄Al₁₄0₂₅:Eu, SrGa₂S₄:A (A = Lanthanoide, Pb), SrGa₂S₄:Pb, PSr₃Gd₂Si₆O₁₈:Pb,Mn, YF₃:Yb,Er, YF₃:Ln (Ln = Lanthanoide), YLiF₄:Ln (Ln = Lanthanoide), Y₃Al₅O₁₂:Ln (Ln = Lanthanoide), YAl₃(BO₄)₃:Nd,Yb, (Y,Ga)BO₃:Eu, (Y,Gd)BO₃:Eu,Y₂Al₃Ga₂O₁₂:Tb, Y₂SiO₅:Ln (Ln = Lanthanoide), Y₂O₃:Ln (Ln = Lanthanoide), Y₂O₂S:Ln (Ln=Lanthanoide), YVO₄:A (A=Lanthanoide, In), Y(P,V)O₄:Eu, YTaO₄:Nb, YAlO₃:A (A = Pr, Tm, Er, Ce), YOCI:Yb,Er, LnPO₄:Ce,Tb (Ln = Lanthanoide oder Gemische von Lanthaniden), LuVO₄:Eu, GdVO₄:Eu, Gd₂O₂S:Tb, GdMgB₅O₁₀:Ce,Tb, LaOBr:Tb, La₂O₂S:Tb, NaGdF₄:Yb,Er, NaLaF₄:Yb,Er, LaF₃:Yb,Er,Tm, BaYF₅:Yb,Er, Ga₂O₃:Dy, GaN:A (A = Pr, Eu, Er, Tm), Bi₄Ge₃O₁₂, LiNbO₃:Nd,Yb, LiNbO₃:Er, LiCaAlF₆:Ce, LiSrAlF₆:Ce, LiLuF₄:A (A = Pr, Tm, Er, Ce), Gd₃Ga₅O₁₂:Tb, Gd₃Ga₅O₁₂:Eu, Li₂B₄O₇:Mn,SiOₓ,Er,Al (0<x<2), YVO₄:Eu, YVO₄:Sm, YVO₄:Dy, LaPO₄:Eu, LaPO₄:Ce, LAPO₄:Ce,Tb, ZnS:Tb, ZnS:TbF₃, ZnS:Eu, ZnS:EuF₃, Y₂O₃:Eu, Y₂O₂S:Eu, Y₂SiO₅:Eu, SiO₂:Dy, SiO₂:Al, Y₂O₃:Tb, ZnS:Tb, ZnS:Ag, ZnS:Cu, Ca₃(PO₄)₂:Eu, Ca₃(PO₄)₂:Eu,Mn, Sr₂SiO₄:Eu, Ba₂SiO₄:Eu, BaAl₂O₄:Eu, MgF₂:Mn, ZnS:Mn, ZnS:Ag, ZnS:Cu, CaSiO₃:A, CaS:A, CaO:A, ZnS:A, Y₂O₃:A, MgF₂:A (A = Lanthanoide), MS, MSe, MTe (M = Zn, Cd, Ge, Sn, Pb), MN, MP, MAs, MSb (M = Al, Ga, In), M₂SiO₄:Eu (M= Ca, Sr, Ba), M₂Si₅N₈:Eu (M = Ca, Sr, Ba), LaSi₃N₅:Ce, Ln₁₋ₓSrₓSi₃₋₂ₓAl₂ₓO₃ₓN₅₋₃ₓ:Ce, LaSi₃N₅:Ce und Ln₁₋ₓSrₓSi₃₋₂ₓAl₂ₓO₃ₓN₅₋₃ₓ:Eu (Ln = Al, Y, La, Lanthanoid) und Kombinationen davon, aufweisen.

12. Verwendung der Leuchtstoffpartikel nach einem der Ansprüche 9 bis 11 zur Beschichtung auf oder zur Einbettung in ein Substrat, wobei das Substrat aus der Gruppe, bestehend aus einem Papier-, Polymer-, Glas-, Metall- oder Keramiksubstrat, ausgewählt ist, beispielsweise zur Diagnostik, Therapie, als Kontrastmittel, als Markierung, Sicherheftsmerkmal, zur Beleuchtung, Datenspeicherung und/oder Datenvervielfältigung.

13. Verfahren zum Herstellen eines beschichteten Substrates, umfassend die Schritte:
(a) das Redispergieren der Leuchtstoffpartikel nach einem der Ansprüche 8 bis 11 in einem Dispersionsmedium,
(b) das Aufbringen der Dispersion auf ein Substrat, und
(c) das Trocknen der auf das Substrat aufgebrachten Dispersion oder das Zulassen, dass die auf das Substrat aufgebrachte Dispersion trocknet.

14. Verfahren nach Anspruch 13 wobei das Dispersionsmedium aus der Gruppe, bestehend aus Aldehyden, Estem, Ethem, Thiolen, wie Dekanthiol, Aromaten, Alkanen, wie Dodekan, Halogenalkanen, Alkoholen, wie Methanol und Ethanol, Polyolen, wie Glycerin und Diethylenglykol, Carbonsäureanhydriden, Aminen, wie Octylamin und Pyridin, Amiden, wie Dimethylformamid, Imiden, Ketonen, Carbon- und Sulfonsäuren, wie Octansäure und Dodecylsulfat, Sulfoxiden, wie Dimethylsulfoxid, Phosphanen, wie Trioctylphosphan (TOP), Phosphanoxiden, wie Trioctylphosphanoxid (TOPO), Phosphaten, wie Octylphosphat, flüssigem Ammoniak, flüssigem Schwefeldioxid, flüssigem Kohlendioxid, Lösungen eines Alkali-, Erdalkali-, Ammonium- und Tetramethylammoniumsalzes, wie Halogeniden, Carbonaten, Hydrogencarbonaten, Sulfaten, Hydrogensulfaten, Nitraten, Acetaten, Oxalaten, Acetylacetonaten, Phosphaten in Wasser, Alkoholen, wie Methanol, Ethanol und Glycerin, Aminen, wie Ethylendiamin, Ethylamin und Pyridin, und Kombinationen davon, ausgewählt ist.

## Claims

1. A method for the targeted synthesis of phosphor particles, which have a particle diameter from 1 to 100 nm, a virtually monodisperse size distribution in the range of ±20%, and, depending on the class of material selected, a quantum yield of at least 20%, comprising the steps of:
(a) reacting at least one metal precursor with at least one precipitating agent in a first solvent or solvent mixture at a temperature in the range from -50°C to 200°C, thus obtaining a first reaction mixture,
(b) thermally treating the first reaction mixture obtained in step (a) at a temperature in the range from 150°C to 700°C for a duration in the range from 5 seconds to 30 minutes in a second solvent or solvent mixture, which comprises at least one ionic liquid, wherein the thermal treatment is performed by means of microwave radiation, and
(c) isolating and/or purifying the phosphor particles,
wherein the at least one metal precursor is selected from the group consisting of metal halides, nitrates, sulfates, hydrogen sulfates, acetates, acetylacetonates, oxalates, carbonates, hydrogen carbonates, phosphates, hydrogen phosphates, dihydrogen phosphates, hybrides, alkoxides, organometallic compounds and combinations thereof, and
wherein the precipitating agent is selected from the group consisting of hydroxides, phosphates, carbonates, oxalates, sulfates, sulfides, halides, ammonia, hydroxylamine, amines, amides, imines, water, and combinations thereof.

2. The method according to claim 1, wherein the at least one ionic liquid is composed of anions and cations, the anions being selected from the group consisting of chloride, bromide, iodide, methanesulfonate, hydrogen sulfate, octyl sulfate, thiocyanate, p-toluolsulfonate, tetrafluoroborate, hexafluorophosphate, bis(pentafluoroethyl)phosphinate, bis[oxalato(2-)]-borate, bis[1,2-benzoldiolato(2-)-O,O']borate, trifluoroacetate, trifluorosulfonate, dicyanamide, tris(trifluoromethylsulfonyl)methide, bis(trifluoromethylsulfonyl)imide, tris(pentafluoroethyl)trifluorophosphate, ethyl sulfate, diethyl phosphate, 2-(2-methoxyethoxy)ethylsulfate, butyl sulfate, and combinations thereof, and the cations being selected from the group consisting of monosubstituted imidazolium derivatives, such as 1-methylimidazolium, disubstituted imidazolium derivatives, such as 1,3-dimethylimidazolium, 1-ethyl-3-methylimidazolium, 1-butyl-3-methylimidazolium, 1-propyl-3-methylimidazolium, 1-hexyl-3-methylimidazolium, 3-methyl-1-octylimidazolium, 1-decyl-3-methylimidazolium, 1-dodecyl-3-methylimidazolium, 3-methyl-1-tetradecylimidazolium, 1-hexadecyl-3-methylimidazolium, 1-octadecyl-3-methylimidazolium, 1-benzyl-3-methylimidazolium, 1-phenylpropyl-3-methylimidazolium, trisubstituted imidazolium derivatives, such as 1,2,3-trimethylimidazolium, 1-ethyl-2,3-dimethylimidazolium, 1-butyl-2,3-dimethylimidazolium, 1-propyl-2,3-dimethylimidazolium, 1-hexyl-2,3-dimethylimidazolium, 1-hexadecyl-2,3-dimethylimidazolium, pyridinium derivatives, such as N-ethylpyridinium, N-butylpyridinium, N-butyl-3,4-dimethylpyridinium, N-butyl-3,5-dimethylpyridinium, N-butyl-3-methylpyridinium, N-butyl-4-methylpyridinium, N-hexylpyridinium, N-octylpyridinium, 1-ethyl-3-hydroxymethylpyridinium, pyrrolidinium derivatieves, such as 1,1-dimethylpyrrolidinium, 1-ethyl-1-methylpyrrolidinium, 1,1-dipropylpyrrolidinium, 1,1-dibutylpyrrolidinium, 1-butyl-1-methylpyrrolidinium, 1,1-dihexylpyrrolidinium, 1-hexyl-1-methylpyrrolidinium, 1-methyl-1-octylpyrrolidinium, phosphonium derivatives, such as tetrabutylphosphonium, trihexyl(tetradecyl)phosphonium, ammonium derivatives, such as tetramethylammonium, tetraethylammonium, tetrabutylammonium, methyltrioctylammonium, ethyl-dimethylpropylammonium, cyclohexyltrimethylammonium, ethanolammonium, guanidinium derivatives, such as guanidinium, N,N,N',N'-tetramethyl-N"-ethylguanidinium, N,N,N',N',N"-pentamethyl-N"-propylguanidinium, N,N,N',N',N"-pentamethyl-N"-isopropylguanidinium, hexamethylguanidinium, isouronium derivatives, such as O-methyl-N,N,N',N'-tetramethylisouronium, S-ethyl-N,N,N',N'-tetramethylisothiouronium, sulfonium derivatives, such as diethylmethethylsulfonium, and combinations thereof.

3. The method according to claim 1 or 2, wherein the first solvent or solvent mixture used in step (a) further comprises at least a first auxiliary solvent selected from the group consisting of aldehydes, esters, ethers, thiols, aromatics, alkanes, haloalkanes, alcohols, such as methanol and ethanol, carboxylic acid anhydrides, amines, amides, such as dimethylformamide, imides, ketones, carboxylic and sulfonic acids, sulfoxides, such as dimethylsulfoxide, liquid ammonia, liquid sulfur dioxide, liquid carbon dioxide, and combinations thereof.

4. The method according to claim 3, which, after the step (a) of reacting at least one metal precursor with at least one precipitating agent, further comprises the step of removing low-boiling auxiliary solvents.

5. The method according to one of claims 1 to 4, wherein the second solvent or solvent mixture used in step (b) comprises at least a second auxiliary solvent selected from the group consisting of aldehydes, esters, ethers, thiols, such as decanthiol, aromatics, alkanes, such as dodecane, haloalkanes, alcohols, polyoles, such as glycerol and diethylglycol, carboxylic acid anhydrides, amines, such as octylamine and pyridine, amides, imides, ketones, carboxylic and sulfonic acids, such as octanoic acid and docecyl sulfate, sulfoxides, phosphines, such as trioctylphosphine (TOP), phosphine oxides, such as trioctylphosphine oxide (TOPO), and phosphates, such as octylphosphate.

6. The method according to one of claims 1 to 5, wherein the thermal treatment in step (b) is performed for a duration of 30 seconds to 600 seconds.

7. The method according to one of claims 1 to 6, wherein the isolation and/or purification of the phosphor particles is performed using a method selected from the group consisting of centrifugation techniques, dialysis techniques, phase transfer techniques, chromatography techniques, ultrafiltration techniques, washing techniques, and combinations thereof.

8. The method according to one of claims 1 to 7, which, prior to or after the step (c) of isolating and/or purifying the phosphor particles, further comprises a step of coating the phosphor particles with an inorganic sheath, thus forming core-shell particles.

9. Inorganic phosphor particles, which have a particle diameter from 1 to 100 nm, a virtually monodisperse size distribution in the range of ±20%, and a quantum yield of at least 20%, obtainable using the method according to one of claims 1 to 8.

10. The inorganic phosphor particles according to claim 9, which are doped with 5 ppm to 50 mol/% of a doping agent, the doping agent being selected from the group consisting of lanthanoides, transition metals, main group elements, and combinations thereof.

11. The inorganic phosphor particles according to claim 9 or 10, which have a chemical composition selected from the group consisting of Lil:Eu, Csl:Na, LiF:Mg, LiF:Mg,Ti, LiF:Mg,Na, KMgF₃:Mn, Al₂O₃:Eu, BaFCI:Eu, BaFCI:Sm, BaFBr:Eu, BaFCl_{0,5}Br_{0,5}:Sm, BaY₂F₈:A (A=Pr, Tm, Er, Ce), BaSi₂O₅:Pb, BaMg₂Al₁₆O₂₇:Eu, BaMgAl₁₃O₂₃:Eu, BaMgAl₁₀O₁₇:Eu, (Ba,Mg)Al₂O₄:Eu, Ba₂P₂O₇:Ti, (Ba,Zn,Mg)₃Si₂O₇:Pb, Ce(Mg,Ba)Al₁₁O₁₉, Ce_{0,60}Tb_{0,35}MgAl₁₀O₁₂, MgAl₁₁O₁₉:Ce,Tb, MgF₂:Mn, MgS:Eu, MgS:Ce, MgS:Sm, MgS:Sm,Ce, (Mg,Ca)S:Eu, MgSiO₃:Mn, 3,5MgOx0,5MgF₂xGeO₂:Mn, MgWO₄:Sm, MgWO₄:Pb, (Zn,Mg)F₂:Mn, (Zn,Be)SiO₄:Mn, Zn₂SiO₄:Mn, ZnO:Zn, ZnO:Zn,Si,Ga, Zn₃(PO₄)₂:Mn, ZnS:A (A=Ag,Al,Cu), (Zn,Cd)S:A (A=Cu,Al,Ag,Ni), CdBO₄:Mn, CaF₂:Mn, CaF₂:Dy; CaS:A (A = lanthanoides, Bi), (Ca,Sr)S:Bi, CaWO₄:Pb, CaWO₄:Sm, CaSO₄:A (A = Mn, lanthanoides), 3Ca₃(PO₄)₂xCa(F,Cl)₂:Sb,Mn, CaSiO₃:Mn,Pb, Ca₂Al₂Si₂O₇:Ce, (Ca,Mg)SiO₃:Ce, (Ca,Mg)SiO₃:Ti, 2Sr_{0,6}(B₂0₃)xSrF₂:Eu, 3Sr₃(PO₄)₂xCaCl₂:Eu, A₃(PO₄)₂xACl₂:Eu (A=Sr,Ca,Ba), (Sr,Mg)₂P₂O₇:Eu, (Sr,Mg)₃(PO4)₂:Sn, SrS:Ce, SrS:Sm,Ce, SrS:Sm, SrS:Eu, SrS:Eu,Sm, SrS:Cu,Ag, Sr₂P₂O₇:Sn, Sr₂P₂O₇:Eu, Sr₄Al₁₄O₂₅:Eu, SrGa₂S₄:A (A = lanthanoides,Pb), SrGa₂S₄:Pb, Sr₃Gd₂Si₆O₁₈:Pb,Mn, YF₃:Yb,Er, YF₃:Ln (Ln = lanthanoides), YLiF₄:Ln (Ln = lanthanoides), Y₃Al₅O₁₂:Ln (Ln = lanthanoides), YAl₃(BO₄)₃:Nd,Yb, (Y,Ga)BO₃:Eu, (Y,Gd)BO₃:Eu, Y₂Al₃Ga₂O₁₂:Tb, Y₂SiO₅:Ln (Ln = lanthanoides), Y₂O₃:Ln (Ln = lanthanoides), Y₂O₂S:Ln (Ln = lanthanoides), YVO₄:A (A = lanthanoides, In), Y(P,V)O₄:Eu, YTaO₄:Nb, YAlO₃:A (A=Pr,Tm,Er,Ce), YOCI:Yb,Er, LnPO₄:Ce,Tb (Ln = lanthanoides or mixtures of lanthanoides, LuVO₄:Eu, GdVO₄:Eu, Gd₂O₂S:Tb, GdMgB₅O₁₀:Ce,Tb, LaOBr:Tb, La₂O₂S:Tb, NaGdF₄:Yb,Er, NaLaF₄:Yb,Er, LaF₃:Yb,Er,Tm, BaYF₅:Yb,Er, Ga₂O₃:Dy, GaN:A (A=Pr,Eu,Er,Tm), Bi₄Ge₃O₁₂, LiNbO₃:Nd,Yb, LiNbO₃:Er, LiCaAlF₆:Ce, LiSrAlF₆:Ce, LiLuF₄:A (A=Pr,Tm,Er,Ce), Gd₃Ga₅O₁₂:Tb, Gd₃Ga₅O₁₂:Eu, Li₂B₄O₇:Mn,SiOx,Er,Al(0<x<2), YVO₄:Eu, YVO₄:Sm, YVO₄:Dy, LaPO₄:Eu, LaPO₄:Ce, LaPO₄:Ce,Tb, ZnS:Tb, ZnS:TbF₃, ZnS:Eu, ZnS:EuF₃, Y₂O₃:Eu, Y₂O₂S:Eu, Y₂SiO₅:Eu, SiO₂:Dy, SiO₂:Al, Y₂O₃:Tb, ZnS:Tb, ZnS:Ag, ZnS:Cu, Ca₃(PO₄)₂:Eu, Ca₃(PO₄)₂:Eu,Mn, Sr₂SiO₄:Eu, Ba₂SiO₄:Eu, BaAl₂O₄:Eu, MgF₂:Mn, ZnS:Mn, ZnS:Ag, ZnS:Cu, CaSiO₃:A, CaS:A, CaO:A, ZnS:A, Y₂O₃:A, MgF₂:A (A = lanthanoides), MS, Mse, MTe (M = Zn, Cd, Cd, Ge, Sn, Pb), MN, MP, Mas, MSb (M = Al, Ga, In), M₂SiO₄:Eu (M = Ca, Sr, Ba), M₂SiO₄:Eu (M = Ca, Sr, Ba), M₂Si₅N₈:Eu (M = Ca, Sr, Ba), LaSi₃N₅:Ce, Ln₁₋ₓSrₓSi₃₋₂ₓAl₂ₓO₃ₓN₅₋₃ₓ:Ce, LaSi₃N₅:Ce, and Ln₁₋ₓSrₓSb₃₋₂ₓAl₂ₓO₃ₓN₅₋₃ₓ: Eu (Ln = Al, Y, La, lanthanoides), and combinations thereof.

12. Use of the phosphor particles according to one of claims 9 to 11, for coating on or embedding in a substrate, the substrate being selected from the group consisting of a paper, polymer, glass, metal, or ceramic substrate, for example for diagnostics, therapy, as a contrast agent, as a marking, safety feature, for illumination, data storage and/or data reproduction.

13. A method for producing a coated substrate, comprising the steps of:
(a) redispersing the phosphor particles according to one of claims 8 to 11 in a dispersion medium,
(b) applying the dispersion to a substrate, and
(c) drying the dispersion applied to the substrate or allowing the dispersion applied to the substrate to dry.

14. The method according to claim 13, wherein the dispersion medium is selected from the group consisting of aldehydes, esters, ethers, thiols, such as decanthiol, aromatics, alkanes, such as dodecane, haloalkanes, alcohols, such as methanol and ethanol, polyoles, such as glycerol and diethylglycol, carboxylic acid anhydrides, amines, such as octylamine and pyridine, amides, such as dimethylformamide, imides, ketones, carboxylic and sulfonic acids, such as octanoic acid and docecyl sulfate, sulfoxides, such as dimethylsulfoxide, phosphines, such as trioctylphosphine (TOP), phosphine oxides, such as trioctylphosphine oxide (TOPO), phosphates, such as octylphosphate, liquid ammonia, liquid sulfur dioxide, liquid carbon dioxide, solutions of an alkali, alkaline-earth, ammonium, and tetramethylammonium salt, such as halides, carbonates, nitrates, hydrogen carbonates, sulfates, hydrogen sulfates, nitrates, acetates, oxalates, acetylacetonates, phosphates in water, alcohols, such as methanol, ethanol, and glycerol, amines, such as ethylenediamine, ethylamine and pyridine, and combinations thereof.

## Revendications

1. Procédé de synthèse ciblée de particules luminescentes, qui présentent un diamètre des particules situé dans l'intervalle allant de 1 à 100 nm, une distribution de tailles presque monodispersée située dans la plage de ±20% et en fonction de la classe de matériau choisie, un rendement quantique d'au moins 20%, comprenant les étapes consistant à :
(a) faire réagir au moins un précurseur métallique avec au moins un réactif de précipitation dans un premier solvant ou mélange de solvants à une température située dans l'intervalle allant de -50°C à 200°C pour obtenir un premier mélange réactionnel ;
(b) traiter thermiquement le premier mélange réactionnel obtenu en (a) à une température située dans l'intervalle allant de 150°C à 700°C pendant une période située dans l'intervalle allant de 5 secondes à 30 minutes dans un deuxième solvant ou mélange de solvants, qui comprend au moins un liquide ionique, où le traitement thermique est réalisé par irradiation de microondes, et
(c) isoler et/ou purifier les particules luminescentes,
où le au moins un précurseur métallique est choisi parmi le groupe consistant en des halogénures, nitrates sulfates, hydrogénosulfates, acétates, acétylacétonates, oxalates, carbonates, hydrogénocarbonates, phosphates, hydrogénophosphates, dihydrogénophosphates, hydrures, alcoolates métalliques, des composés métalloorganiques et leurs combinaison, et
où le réactif de précipitation est choisi parmi le groupe consistant en des hydroxydes, phosphates, carbonates, oxalates, sulfates, sulfures, halogénures, l'ammoniaque, l'hydroxylamine, des amines, amides, imines, l'eau et leurs combinaisons.

2. Procédé selon la revendication 1, où le au moins un liquide ionique est composé de cations et d'anions, où les anions sont choisis parmi le groupe consistant en chlorure, bromure, iodure, méthanesulfonate, hydrogénosulfate, octylsulfate, p-toluènesulfonate, tétrafluoroborate, hexafluorophosphate, bis(pentafluoroéthyl)phosphinate, bis[oxalato(2-)]borate, bis[1,2-benzènediolato(2-)-O,O']borate, trifluoroacétate, trifluorosulfonate, dicyanamide, tris(trifluorométhylsulfonyl)méthide, bis(trifluorométhylsulfonyl)imide, tris(pentafluoroéthyl)trifluorophosphate, éthylsulfate, diéthylphosphate, 2-(2-méthoxyéthoxy)éthylsulfate, butylsulfate et leurs combinaisons, et les cations sont choisis parmi le groupe consistant en des dérivés imidazolium monosubstitués, comme 1-méthylimidazolium, des dérivés imidazolium disubstitués, comme 1,3-diméthylimidazolium, 1-éthyl-3-méthylimidazolium, 1-butyl-3-méthylimidazolium, 1-propyl-3-méthylimidazolium, 1-hexyl-3-méthylimidazolium, 3-méthyl-1-octylimidazolium, 1-décyl-3-méthylimidazolium, 1-dodécyl-3-méthylimidazolium, 3-méthy-1-tétradécylimidazolium, 1-hexadécyl-3-méthylimidazolium, 1-octadécyl-3-méthylimidazolium, 1-benzyl-3-méthylimidazolium, 1-phénylpropyl-3-méthylimidazolium, des dérivés imidazolium trisubstitués, comme 1,2,3-triméthylimidazolium, 1-éthyl-2,3-diméthylimidazolium, 1-butyl-2,3-diméthylimidazolium, 1-propyl-2,3-diméthylimidazolium, 1-hexyl-2,3-diméthylimidazolium, 1-hexadécyl-2,3-diméthylimidazolium, des dérivés pyridinium, comme N-éthylpyridinium, N-butylpyridinium, N-butyl-3,4-diméthylpyridinium, N-butyl-3,5-diméthylpyridinium, N-butyl-3-méthylpyridinium, N-butyl-4-méthylpyridinium, N-hexylpyridinium, N-octylpyridinium, 1-éthyl-3-hydroxyméthylpyridinium, des dérivés pyrrolidinium, comme 1,1-diméthylpyrrolidinium, 1-éthyl-1-méthylpyrrolidinium, 1,1-dipropylpyrrolidinium, 1,1-dibutylpyrrolidinium, 1-butyl-1-méthylpyrrolidinium, 1,1-dihexylpyrrolidinium, 1-hexyl-1-méthylpyrrolidinium, 1-méthyl-1-octylpyrrolidinium, des dérivés phosphonium, comme tétrabutylphosphonium, trihexyl(tétradécyl)phosphonium, des dérivés ammonium, comme tétraméthylammonium, tétraéthylammonium, tétrabutylammonium, méthyltrioctylammonium, éthyldiméthylpropylammonium, cyclohexyltriméthylammonium, éthanolammonium, des dérivés guanidinium, comme guanidinium, N,N,N',N'-tétraméthyl-N"-éthylguanidinium, N,N,N',N',N"-pentaméthyl-N"-propylguanidinium, N,N,N',N',N"-pentaméthyl-N"-isopropylguanidinium, hexaméthylguanidinium, des dérivés isouronium, comme O-méthyl-N,N,N',N'-tétraméthylisouronium, S-éthyl-N,N,N',N'-tétraméthylisothiouronium, des dérivés sulfonium, comme diéthylméthyléthylsulfonium, et leurs combinaisons.

3. Procédé selon la revendication 1 ou 2, où le premier solvant ou mélange de solvants utilisé à l'étape (a) comprend en outre, au moins un premier co-solvant, choisi parmi le groupe consistant en des aldéhydes, des esters, des éthers, des thiols, des aromatiques, des alcanes, des halogénoalcanes, des alcools, comme le méthanol et l'éthanol, des anhydrides d'acide carboxylique, des amines, des amides, comme le diméthylformamide, des imides, des cétones, des acides carboxyliques et sulfoniques, des sulfoxydes, comme le diméthylsulfoxyde, l'ammoniac liquide, le dioxyde de soufre liquide, le dioxyde de carbone liquide, et leurs combinaisons.

4. Procédé selon la revendication 3, qui comprend après l'étape (a) de réaction d'au moins un précurseur métallique avec au moins un réactif de précipitation, l'étape d'élimination du co-solvant de faible point d'ébullition.

5. Procédé selon l'une des revendications 1 à 4, où le deuxième solvant ou mélange de solvants utilisé à l'étape (b) comprend en outre, au moins un deuxième co-solvant, choisi parmi le groupe consistant en des aldéhydes, des esters, des éthers, des thiols, comme le décanethiol, des aromatiques, des alcanes, comme le dodécane, des halogénoalcanes, des alcools, des polyols, comme le glycérol et le diéthylèneglycol, des anhydrides d'acide carboxylique, des amines, comme l'octylamine et la pyridine, des amides, des imides, des cétones, des acides carboxyliques et sulfoniques, comme l'acide octanoïque et le sulfate de dodécyle, des sulfoxydes, des phosphanes, comme le trioctylphosphane (TOP), des phosphanoxydes, comme le trioctylphoshanoxyde (TOPO), et des phosphates, comme le phosphate d'octyle.

6. Procédé selon l'une des revendications 1 à 5, où le traitement thermique de l'étape (b) est réalisé pendant une période allant de 30 secondes à 600 secondes.

7. Procédé selon l'une des revendications 1 à 6, où l'isolement et/ou la purification des particules luminescentes sont réalisés par un procédé choisi parmi le groupe consistant en les techniques de centrifugation, les techniques de dialyse, les techniques de transfert de phase, les techniques de chromatographie, les techniques d'ultrafiltration, les techniques de lavage et leurs combinaisons.

8. Procédé selon l'une des revendications 1 à 7, qui comprend avant ou après l'étape (c) d'isolement et/ou de purification des particules luminescentes, une étape de revêtement des particules luminescentes avec une coque inorganique avec formation de particules coeur-enveloppe.

9. Particules luminescentes inorganiques ayant un diamètre de particule situé dans l'intervalle allant de 1 à 100 nm, une distribution de tailles presque monodispersée dans la plage de ±20% et un rendement quantique d'au moins 20%, pouvant être obtenues par le procédé selon l'une des revendications 1 à 8.

10. Particules luminescentes inorganiques selon la revendication 9, dopées avec 5 ppm à 50% en moles d'agent dopant, où l'agent dopant est choisi parmi le groupe consistant en les lanthanides, les métaux de transition, des éléments des groupes principaux et leurs combinaisons.

11. Particules luminescentes inorganiques selon la revendication 9 ou 10, qui présentent une composition chimique choisie parmi le groupe consistant en Lil:Eu, Csl:Na, LiF:Mg, LiF:Mg,Ti, LiF:Mg,Na, KMgF₃:Mn, Al₂O₃:Eu, BaFCl:Eu, BaFCI:Sm, BaFBr:Eu, BaFCl_{0,5}Br_{0,5}:Sm, BaY₂F₈:A (A= Pr, Tm, Er, Ce), BaSi₂O₅:Pb, BaMg₂Al₁₆O₂₇:Eu, BaMgAl₁₃O₂₃:Eu, BaMgAl₁₀O₁₇:Eu, (Ba,Mg)Al₂O₄:Eu, Ba₂P₂O₇:Ti, (Ba,Zn,Mg)₃Si₂0O₇:Pb , Ce(Mg,Ba)Al₁₁O₁₉, Ce_{0,60}Tb_{0,35}MgAl₁₀O₁₉, MgAl₁₁O₁₉:Ce,Tb, MgF₂:Mn, MgS:Eu, MgS:Ce, MgS:Sm, . MgS:Sm,Ce, (Mg,Ca)S:Eu, MgSiO₃:Mn, 3,5MgOx0,5MgF₂xGeO₂:Mn, MgWO₄:Sm, MgWO₄:Pb, (Zn,Mg)F₂:Mn, (Zn,Be)SiO₄:Mn, Zn₂SiO₄:Mn, ZnO:Zn, ZnO:Zn,Si,Ga, Zn₃(PO₄)₂:Mn, ZnS:A, (A= Ag, Al, Cu), (Zn,Cd)S:A (A = Cu, Al, Ag, Ni), CdBO₄:Mn, CaF₂:Mn, CaF₂:Dy, CaSO₄:A (A = lanthanide, Bi), (Ca,Sr)S:Bi, CaWO₄:Pb,·CaWO₄:Sm,·CaSO₄:A (A = Mn, lanthanide), 3Ca₃(PO₄)₂XCa(F,Cl)₂:Sb,Mn, CaSiO₃:Mn,Pb, Ca₂Al₂Si₂O₇:Ce, (Ca,Mg)SiO₃:Ce, (Ca,Mg)SiO₃:Ti, 2Sr_{0,6}(B₂O₃)xSrF₂:Eu, 3Sr₃(PO₄)₂xCaCl₂:Eu, A₃(PO₄)₂xACl₂:Eu (A = Sr, Ca, Ba), (Sr,Mg)₂P₂O₇:Eu, (Sr,Mg)₃(PO₄)₂:Sn, SrS:Ce, SrS:Sm,Ce, SrS:Sm, SrS:Eu, SrS:Eu,Sm, SrS:Cu,Ag, Sr₂P₂O₇:Sn, Sr₂P₂O₇:Eu, Sr₄Al₁₄O₂₅:Eu. SrGa₂S₄:A(A = lanthanide, Pb), SrGa₂S₄:Pb, Sr₃Gd₂Si₆O₁₈:Pb,Mn, YF₃:Yb,Er, YF₃:Ln . (Ln = lanthanide), YLiF₄:Ln (Ln = lanthanide), Y₃Al₅O₁₂:Ln (Ln = lanthanide); YAl₃(BO₄)₃:Nd,Yb, (Y,Ga)BO₃:Eu, (Y,Gd)BO₃:Eu, Y₂Al₃Ga₂O₁₂:Tb, Y₂SiO₅:Ln (Ln = lanthanide), Y₂O₃:Ln (Ln = lanthanide), Y₂O₂S:Ln (Ln = lanthanide), YVO₄:A (A= lanthanide, In), Y(P,V)O₄:Eu, YTaO₄:Nb, YAlO₃:A (A = Pr, Tm, Er, Ce), YOCI:Yb,Er, LnPO₄:Ce,Tb (Ln = lanthanide ou mélange de lanthanides), LuVO₄:Eu, GdVO₄:Eu, Gd₂O₂S:Tb, GdMgB₅O₁₀:Ce,Tb, LaOBr:Tb;·La₂O₂S:Tb,·NaGdF₄:Yb,Er, NaLaF₄:Yb,Er, LaF₃:Yb,Er,Tm, BaYF₅:Yb,Er, Ga₂O₃:Dy, GaN:A (A = Pr, Eu, Er, Tm), Bi₄Ge₃O₁₂, LiNbO₃:Nd,Yb, LiNbO₃:Er, LiCaAlF₆:Ce, LiSrAlF₆:Ce, LiLuF₄:A (A = Pr, Tm, Er, Ce), Gd₃Ga₅O₁₂:Tb,·Gd₃Ga₅O₁₂:Eu, Li₂B₄O₇:Mn,SiOₓ,Er,Al (0<x<2), YVO₄:Eu, YVO₄:Sm, YVO₄:Dy, LaPO₄:Eu, LaPO₄:Ce, LaPO₄:Ce,Tb, ZnS:Tb, ZnS:TbF₃, ZnS:Eu, ZnS:EuF₃, Y₂O₃:Eu, Y₂O₂S:Eu, . Y₂SiO₅:Eu, SiO₂:Dy, SiO₂:Al, Y₂O₃:Tb, ZnS:Tb, ZnS:Ag, ZnS:Cu, Ca₃(PO₄)₂:Eu, Ca₃(PO₄)₂:Eu,Mn, Sr₂SiO₄:Eu, Ba₂SiO₄:Eu, BaAl₂O₄:Eu, MgF₂:Mn, ZnS:Mn, ZnS:Ag, ZnS:Cu, CaSiO₃:A, CaS:A, CaO:A, ZnS:A, Y₂O₃:A, MgF₂:A (A= lanthanide), MS, MSe, MTe (M = Zn, Cd, Ge, Sn, Pb), MN, MP, MAs, MSb (M = Al, Ga, In), M₂SiO₄:Eu (M= Ca, Sr, Ba), M₂Si₅N₈:Eu (M = Ca, Sr, Ba), LaSi₃N₅:Ce, Ln₁₋ₓSrₓSi₃₋₂ₓAl₂ₓO₃ₓN₅₋₃ₓ:Ce, . LaSi₃N₅:Ce et Ln₁₋ₓSrₓSi₃₋₂ₓAl₂ₓO₃ₓN₅₋₃ₓ:Eu (Ln = Al, Y, La, lanthanide) et leurs combinaisons.

12. Utilisation des particules luminescentes selon l'une des revendications 9 à 11, pour le revêtement ou pour l'intégration dans un substrat, où le substrat est choisi dans le groupe consistant en un substrat en papier, en polymère, en verre, en métal ou en céramique, par exemple pour un diagnostic, une thérapie, comme agent de contraste, comme marquage, caractéristique de sécurité, pour l'éclairage, le stockage de données et/ou la reproduction de données.

13. Procédé de préparation d'un substrat revêtu, comprenant les étapes consistant à :
(a) redisperser les particules luminescentes selon l'une des revendications 8 à 11 dans un milieu de dispersion,
(b) appliquer la dispersion sur un substrat, et
(c) sécher la dispersion appliquée sur le substrat ou laisser au repos de sorte que la dispersion appliquée sur le substrat sèche.

14. Procédé selon la revendication 13, où le milieu de dispersion est choisi parmi le groupe consistant en des aldéhydes, des esters, des éthers, des thiols, comme le décanethiol, des aromatiques, des alcanes, comme le dodécane, des halogénoalcanes, des alcools, comme le méthanol et l'éthanol, des polyols, comme le glycérol et le diéthylèneglycol, des anhydrides d'acide carboxylique, des amines, comme l'octylamine et la pyridine, des amides, comme le diméthylformamide, des imides, des cétones, des acides carboxyliques et sulfoniques, comme l'acide octanoïque et le sulfate de dodécyle, des sulfoxydes, comme le diméthylsulfoxyde, des phosphanes, comme le trioctylphosphane (TOP), des phosphanoxydes, comme le trioctylphoshanoxyde (TOPO), des phosphates, comme le phosphate d'octyle, l'ammoniac liquide, le dioxyde de soufre liquide, le dioxyde de carbone liquide, des solutions d'un sel alcalin, alcalino-terreux, d'ammonium ou de tétraméthylammonium, comme des halogénures, des carbonates, des hydrogénocarbonates, des sulfates, des hydrogénosulfates, des nitrates, des acétates, des oxalates, des acétylacétonates, des phosphates dans l'eau, des alcools, comme le méthanol, l'éthanol et le glycérol, des amines, comme l'éthylènediamine, l'éthylamine et la pyridine, et leurs combinaisons.
